# FASCICULE DE BREVET EUROPEEN

(11) **EP 3 949 826 B1**
(45) Date de publication et mention de la délivrance du brevet: **25.10.2023**
(21) Numéro de dépôt: 21188303.8
(22) Date de dépôt: 28.07.2021
(51) Int. Cl.: A47L 23/02, A61L 2/18

(54) **PLATEFORME POUR LA DESINFECTION DE SEMELLES DE CHAUSSURES PAR PULVERISATION D'UN LIQUIDE DESINFECTANT SANS RINCAGE**
PLATTFORM FÜR DIE DESINFEKTION VON SCHUHSOHLEN DURCH VERSPRÜHEN EINER DESINFEKTIONSFLÜSSIGKEIT OHNE ABSPÜLEN
PLATFORM FOR DISINFECTION OF SOLES OF SHOES BY SPRAYING A NO-RINSE DISINFECTANT LIQUID

(30) Priorité: 03.08.2020 FR 2008228
(43) Date de publication de la demande: 09.02.2022
(73) Titulaire: Acemia Industrie, 44118 La Chevrolière (FR)
(72) Inventeur: TALBOT, Jérôme, 44120 VERTOU (FR)
(74) Mandataire: Plasseraud IP

(56) Documents cités:
- WO-A1-02/21991
- CN-U- 203 694 167
- JP-A- 2012 232 028
- KR-A- 20140 137 900
- KR-Y1- 200 457 168
- US-A1- 2017 035 918

## Description

### Domaine technique

La présente invention est relative à une plateforme pour la désinfection des chaussures, bottes ou sabots d'un utilisateur par projection d'un liquide désinfectant, ainsi qu'à un procédé de désinfection des chaussures d'un utilisateur par projection d'un liquide désinfectant susceptible d'être mis en oeuvre avec une telle plateforme.

Le domaine de l'invention est celui de la désinfection des chaussures, bottes, sabots ou similaire, réalisé à une échelle industrielle, par exemple pour des employés de sites industriels, préalablement à leur entrée dans une zone de production.

### Technique antérieure

En effet, pour certaines applications industrielles, nécessitant un haut degré de salubrité des employés lors de la production, par exemple dans l'industrie agroalimentaire, il est prévu de désinfecter, éventuellement après un nettoyage préalable, les chaussures des employés entrant sur le site de production, afin d'éliminer les bactéries présentes sur lesdites chaussures, bottes, sabots ou similaires et notamment les semelles, en contact avec le sol, de sorte à éviter un risque de contamination des produits réalisés sur ledit site de production.

Afin de s'assurer qu'une telle désinfection est réalisée selon une procédure déterminée pour chacun des employés pénétrant sur ledit site de production, et avec un temps d'exécution réduit, il a ainsi été développé des plateformes pour la désinfection des semelles de chaussures, sur lesquelles viennent se positionner les employés, avant qu'un cycle de désinfection, et éventuellement de nettoyage, soit appliqué à leurs chaussures.

De façon connue, une telle plateforme de désinfection des chaussures comporte généralement un support sur lequel un utilisateur se positionne et des moyens de projection de liquide désinfectant, configuré pour projeter du liquide désinfectant sur les chaussures de l'utilisateur. De telles plateformes sont par exemple connues des documents WO02/21991A1, JP2012232028, US2017/035918A1, KR20140137900A.

Les plateformes de désinfection des chaussures de l'art antérieur emploient généralement un liquide désinfectant mélangé avec de l'eau sanitaire, projeté sous forme d'un ou plusieurs jets, ce qui présente plusieurs inconvénients.

En effet, pour assurer l'alimentation en eau sanitaire de la plateforme, celle-ci est reliée au réseau d'eau sanitaire, ce qui nécessite de positionner la plateforme à proximité d'une arrivée d'eau sanitaire d'un réseau de distribution d'eau sanitaire.

Par ailleurs, et selon les constations de l'inventeur, l'emploi d'eau mélangé au liquide désinfectant et leur projection sous forme d'un ou plusieurs jets, génère des projections de liquide à proximité de la plateforme, accentué par des mouvements mécaniques complémentaires en cas de nettoyage mécanique associé, lesquelles peuvent représenter un vecteur de propagation des bactéries.

Selon les constations de l'inventeur, l'emploi d'un nettoyage mécanique associé augmente également la quantité de liquide désinfectant consommé pour réaliser la désinfection des chaussures d'un utilisateur.

Egalement, le liquide désinfectant mélangé à l'eau présente un temps de séchage important, ce qui nécessite, soit de prévoir un temps de séchage des chaussures de l'utilisateur sur la plateforme, pendant le cycle de désinfection, ce qui augmente sensiblement la durée d'un tel cycle de désinfection et donc réduit la cadence de désinfection des employés avant leur pénétration dans la zone de production, soit présente le risque que les employés, après leur passage sur la plateforme, transportent du liquide sur le site de production et risquent de former des flaques dans lesquelles des bactéries vont pouvoir se développer.

### Problème technique

L'objectif de l'invention est donc de pallier les inconvénients des plateformes de désinfection des chaussures de l'art antérieur en proposant une plateforme de désinfection des chaussures ne nécessitant pas d'être raccordée à un réseau de distribution d'eau.

Un autre objectif de la présente invention est de proposer une telle plateforme réduisant les risques de propagation des bactéries dans une zone de production à l'entrée de laquelle elle est positionnée.

Un autre objectif de la présente invention est de proposer une telle plateforme permettant de réaliser plus rapidement une désinfection des chaussures d'un utilisateur.

Un autre objectif de la présente invention est de proposer une telle plateforme permettant de réduire la quantité de liquide désinfectant consommée pour réaliser la désinfection des chaussures d'un utilisateur.

Un autre objectif de la présente invention est de proposer une telle plateforme de conception simple de coût de revient et de coût de fonctionnement réduits.

D'autres buts et avantages de la présente invention apparaîtront au cours de la description qui va suivre qui n'est donnée qu'à titre indicatif et qui n'a pas pour but de la limiter.

### Exposé de l'invention

Il est proposé une plateforme pour la désinfection des chaussures d'un utilisateur par projection d'un liquide désinfectant, comportant :
- un châssis,
- un support configuré pour recevoir les chaussures d'un utilisateur, et solidaire du châssis,
- un moyen de projection de liquide désinfectant, configuré pour projeter du liquide désinfectant sur les chaussures de l'utilisateur, et solidaire du châssis,
- une unité de commande reliée au moyen de projection, et configurée de sorte à commander la projection de liquide désinfectant par le moyen de projection.

Selon l'invention, le support comporte une grille présentant une pluralité d'ouvertures, la grille étant configurée de sorte à recevoir en appui la face inférieure des semelles de chaussures de l'utilisateur.

Selon l'invention, le moyen de projection présente une pluralité de buses, disposées sous la grille du support, et configurées de sorte à projeter du liquide désinfectant sur les chaussures de l'utilisateur au travers au moins une partie des ouvertures de la grille du support, sous forme de nébulisation, selon une quantité déterminée, commandée par l'unité de commande.

Selon l'invention:
- le moyen de projection comporte un réservoir de liquide désinfectant, solidaire du châssis, chaque buse étant reliée au réservoir par l'intermédiaire d'une pompe et d'une électrovanne, intercalée entre la pompe et au moins une buse ;
- le moyen de projection comporte une unique pompe configurée de sorte à alimenter simultanément l'ensemble des buses, et chaque buse est reliée à une électrovanne distincte, intercalée entre la pompe et chacune des buses.

Selon des caractéristiques optionnelles:
- la pompe est configurée de sorte à fournir un débit de liquide désinfectant compris entre 7L/heure et 20 L/heure, de préférence compris entre 10 L/heure et 15 L/heure ;
- chaque buse est configurée pour projeter du liquide désinfectant sous forme d'une nébulisation de particules de diamètre compris entre 40 µm et 300 µm, de préférence entre 80 µm et 150 µm ;
- chaque buse est configurée pour projeter du liquide désinfectant sous forme d'une nébulisation lorsque alimentée en liquide désinfectant avec un débit compris entre 0,05 L/min et 1,2 L/min, de préférence entre 0,07 L/min et 0,09 L/min ;
- le moyen de projection comprend quatre buses identiques, formant deux paires de buses, avec une première paire de buses positionnée de sorte à projeter du liquide désinfectant sur la face inférieure de la semelle d'une première chaussure d'un utilisateur, et une deuxième paire de buses positionnées de sorte à projeter du liquide désinfectant sur la face inférieure de la semelle d'une deuxième chaussure de l'utilisateur ;
- les buses de chaque paire de buses sont disposées en vis-à-vis l'une de l'autre ;
- les deux paires de buses sont symétriques entre elles ;
- le liquide désinfectant consiste en un peroxyde ;
- le peroxyde est le peroxyde d'Hydrogène ;
- les ouvertures de la grille présentent, au moins pour une partie d'entre elles, une forme sensiblement rectangulaire de longueur, selon la direction transversale de la plateforme, comprise entre 50 mm et 80 mm, de préférence comprise entre 60 mm et 75 mm, et de largeur, selon la direction longitudinale de la plateforme, comprise entre 50 mm et 80 mm, de préférence entre 60 mm et 75 mm, deux ouvertures adjacentes étant séparées entre elles d'une épaisseur de matière comprise entre 3 mm et 6mm, de préférence comprise entre 4 mm et 5 mm;
- la grille comporte une pluralité de fils métalliques soudés entre eux, formant lesdites ouvertures, le diamètre des fils étant compris entre 3 mm et 6mm, de préférence compris entre 4 mm et 5 mm;
- le support comporte un élément de séparation des pieds d'un utilisateur, fixé sur le support, de préférence sensiblement au milieu de la grille, selon la direction transversale de la plateforme, et configuré de sorte délimiter sur le support, selon la direction transversale de la plateforme, un premier espace configuré pour recevoir la chaussure droite d'un utilisateur et un deuxième espace configuré pour recevoir la chaussure gauche d'un utilisateur ;
- le support comporte :
   -- une paroi de butée droite, configurée pour délimiter en combinaison avec l'élément de séparation le premier espace du support, selon la direction transversale de la plateforme, et/ou
- une paroi de butée avant droite et une paroi de butée arrière droite, configurées pour délimiter le premier espace du support, selon la direction longitudinale de la plateforme, respectivement à l'avant et à l'arrière, et/ou
   -- une paroi de butée gauche, configurée pour délimiter en combinaison avec l'élément de séparation le deuxième espace du support, selon la direction transversale de la plateforme, et/ou
   -- une paroi de butée avant gauche et une paroi de butée arrière gauche, configurées pour délimiter le deuxième espace du support, selon la direction longitudinale de la plateforme, respectivement à l'avant et à l'arrière ;
- une première partie des buses sont configurées de sorte à projeter du liquide désinfectant uniquement dans le premier espace du support et une deuxième partie des buses sont configurées de sorte à projeter du liquide désinfectant uniquement dans le deuxième espace du support ;
- la plateforme comprend en outre un moyen de détection des chaussures d'un utilisateur, relié à l'unité de commande, configuré de sorte à détecter la présence des chaussures d'un utilisateur sur la grille du support, l'unité de commande étant configurée pour commander la projection de liquide désinfectant sur les chaussures d'un utilisateur par le moyen de projection après la détection de la présence des deux chaussures de l'utilisateur sur la grille du support ;
- le moyen de détection est configuré pour détecter la présence de la chaussure droite d'un utilisateur dans le premier espace du support et pour détecter la présence de la chaussure gauche de l'utilisateur dans le deuxième espace du support, et l'unité de commande est configurée pour commander la projection de liquide désinfectant sur les chaussures d'un utilisateur par le moyen de projection après la détection de la présence de la chaussure droite de l'utilisateur dans le premier espace du support et de la présence de la chaussure gauche de l'utilisateur dans le deuxième espace du support ;
- le moyen de détection comporte un moyen d'émission d'un signal, par exemple un signal lumineux, de préférence infrarouge, et un moyen de réception du signal émis par le moyen d'émission ;
- le moyen de détection comporte :
   -- un premier moyen d'émission, configuré pour émettre un signal dans le premier espace du support, et un premier moyen de réception, configuré pour recevoir le signal émis par le premier moyen d'émission, et
   -- un deuxième moyen d'émission, configuré pour émettre un signal dans le deuxième espace du support, et un deuxième moyen de réception, configuré pour recevoir le signal émis par le deuxième moyen d'émission.

Selon l'invention, ladite unité de commande peut être est configurée de sorte à commander la projection de liquide désinfectant par le moyen de projection, par le lancement d'un cycle de désinfection avec projection de liquide désinfectant sous forme de nébulisation sur les chaussures d'un utilisateur, par les buses du moyen de projection au travers des ouvertures de la grille du support, sur lequel est reçue en appui la face inférieure des semelles de chaussures de l'utilisateur et dans laquelle l'étape de lancement du cycle de désinfection comportant les sous étapes successives :
- activation préalable de la pompe pendant une durée comprise entre 0,5 secondes et 2 secondes, de préférence environ 1 seconde, les électrovannes demeurant fermées,
- ouverture des électrovannes et maintien de la pompe activée pendant une durée comprise entre 2 secondes et 5 secondes, de préférence entre 3 et 4 secondes, de sorte à projeter du liquide désinfectant au travers des ouvertures de la grille du support par l'intermédiaire des buses.

Toujours selon l'invention, le débit de la pompe est bien inférieur au débit des buses , et dans laquelle l'activation de la pompe préalablement à l'ouverture des électrovannes pendant une durée prédéterminée, comprise entre 0,5 secondes et 2 secondes, de préférence environ 1 seconde, est configurée pour mettre en pression le circuit d'alimentation des buses, de sorte que la pression y soit suffisante pour assurer la projection de liquide désinfectant sous forme d'une nébulisation et pendant ladite durée d'activation de la pompe comprise entre 2 secondes et 5 secondes, de préférence entre 3 et 4 secondes, en fournissant le débit d'alimentation en liquide désinfectant souhaité aux buses , par exemple compris entre 0,05 L/min et 1,2 L/min, de préférence entre 0,07 L/min et 0,09 L/min, et dans laquelle au-delà de cette durée d'activation de la pompe comprise entre 2s et 4s.

Par ailleurs, le débit fourni par la pompe est déterminé insuffisant pour que les buses puissent projeter du liquide désinfectant sous forme d'une nébulisation, de sorte que celui-ci est projeté desdites buses sous forme d'un jet à faible pression, lequel sous l'effet de la gravité, retombe intégralement sous la grille du support, et est éventuellement intégralement reçu dans le bac de récupération et l'ouverture d'évacuation du châssis, et sans passer au travers des ouvertures de ladite grille.

Ainsi, même en cas de dysfonctionnement, grâce à une telle configuration de la pompe et des buses, après la durée déterminée de projection de produit désinfectant sous forme de nébulisation par lesdites buses 41, le liquide désinfectant ne risque pas d'être projeté au-delà du châssis 2 de la plateforme, ce qui supprime les risques de formation de flaques sur le sol à proximité de la plateforme

L'invention concerne enfin un procédé de désinfection des chaussures d'un utilisateur mis en oeuvre avec une plateforme selon l'un des modes de réalisation de l'invention, comprenant l'étape :
- lancement d'un cycle de désinfection avec projection de liquide désinfectant sous forme de nébulisation sur les chaussures d'un utilisateur, par les buses du moyen de projection au travers des ouvertures de la grille du support, sur lequel est reçu en appui la face inférieure des semelles de chaussures de l'utilisateur.

Selon un mode de réalisation du procédé, ledit procédé comprend en outre, préalablement à l'étape de lancement du cycle de désinfection, une étape de détection de la présence des chaussures d'un utilisateur sur la grille du support, l'étape de lancement du cycle de désinfection n'étant exécutée que si la présence des chaussures de l'utilisateur sur la grille du support est détectée.

Selon un mode de réalisation du procédé, l'étape de lancement du cycle de désinfection comporte les sous étapes successives :
- activation de la pompe pendant une durée comprise entre 0,5 secondes et 2 secondes, de préférence environs 1 seconde, les électrovannes demeurant fermées,
- ouverture des électrovannes et maintien de la pompe activée pendant une durée comprise entre 2 secondes et 5 secondes, de préférence entre 3 et 4 secondes, de sorte à projeter du liquide désinfectant au travers des ouvertures de la grille du support par l'intermédiaire des buses.

En particulier et avantageusement, le débit de la pompe est déterminé bien inférieur au débit des buses, et dans lequel l'activation de la pompe préalablement à l'ouverture des électrovannes pendant une durée prédéterminée, comprise entre 0,5 secondes et 2 secondes, de préférence environ 1 seconde, est configurée pour mettre en pression le circuit d'alimentation des buses , de sorte que la pression y soit suffisante pour assurer la projection de liquide désinfectant sous forme d'une nébulisation et pendant ladite durée d'activation de la pompe comprise entre 2 secondes et 5 secondes, de préférence entre 3 et 4 secondes, en fournissant le débit d'alimentation en liquide désinfectant souhaité aux buses, par exemple compris entre 0,05 L/min et 1,2 L/min, de préférence entre 0,07 L/min et 0,09 L/min, et dans lequel au-delà de cette durée d'activation de la pompe comprise entre 2s et 4s, le débit fourni par la pompe est insuffisant pour que les buses puissent projeter du liquide désinfectant sous forme d'une nébulisation, de sorte que celui-ci est projeté desdites buses sous forme d'un jet à faible pression, lequel sous l'effet de la gravité, retombe intégralement sous la grille du support, et est éventuellement intégralement reçu dans le bac de récupération et l'ouverture d'évacuation du châssis, et sans passer au travers des ouvertures de ladite grille.

### Brève description des dessins

D'autres caractéristiques, détails et avantages de l'invention apparaîtront à la lecture de la description détaillée ci-après, et à l'analyse des dessins annexés, sur lesquels :
**Fig. 1**

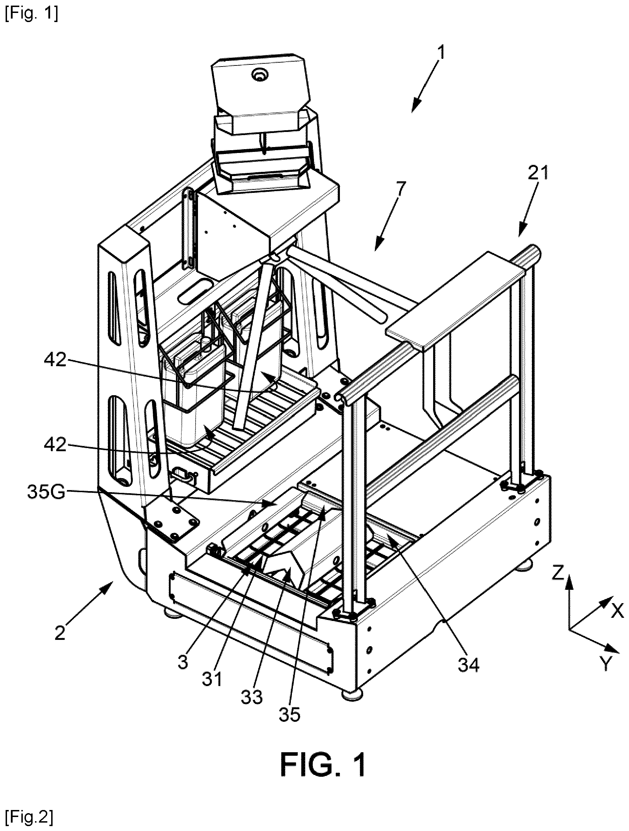

   [Fig. 1] représente une vue en perspective d'une plateforme de désinfection selon un mode de réalisation conforme à l'invention.
**Fig. 2**

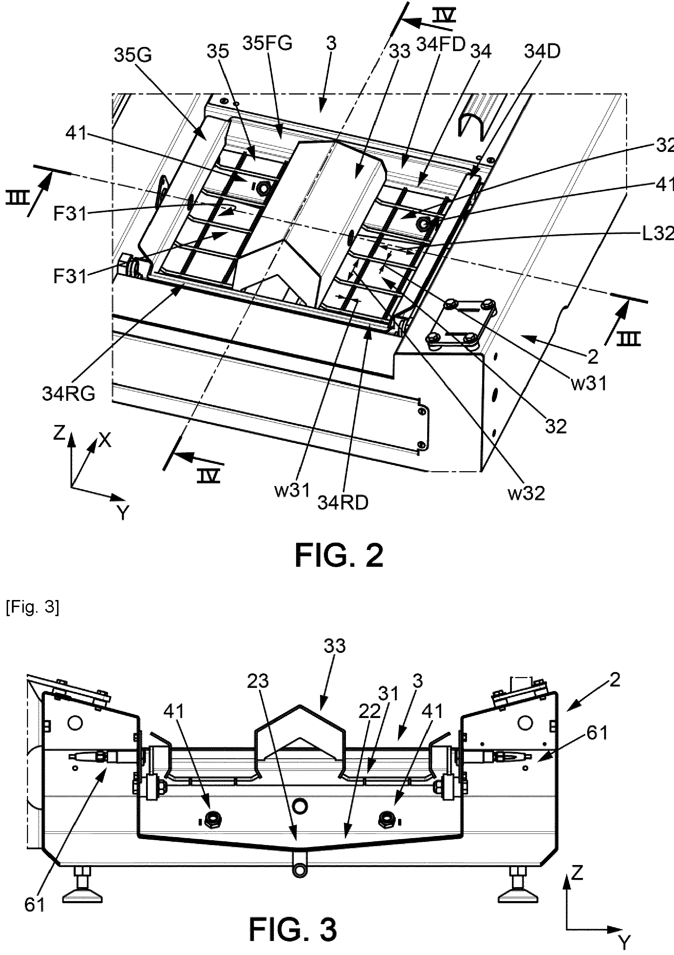

   [Fig. 2] représente une vue de détail en perspective du support de la plateforme de la figure 1.
**Fig. 3**
   [Fig. 3] représente une vue en coupe selon la ligne III-III de la figure 2.
**Fig. 4**

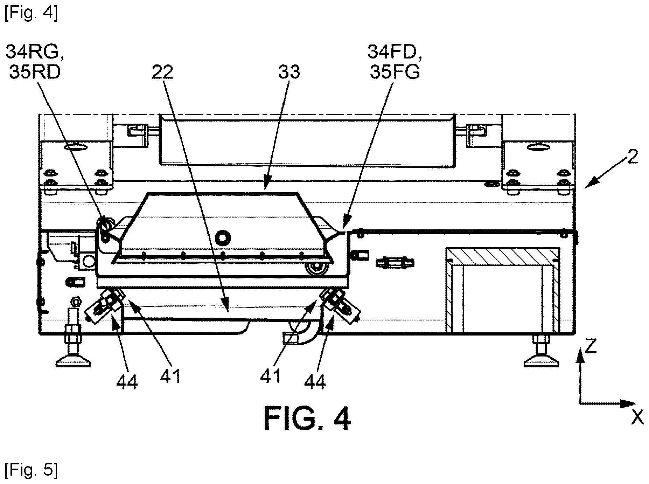

   [Fig. 4] représente une vue en coupe selon la ligne IV-IV de la figure 2.
**Fig. 5**

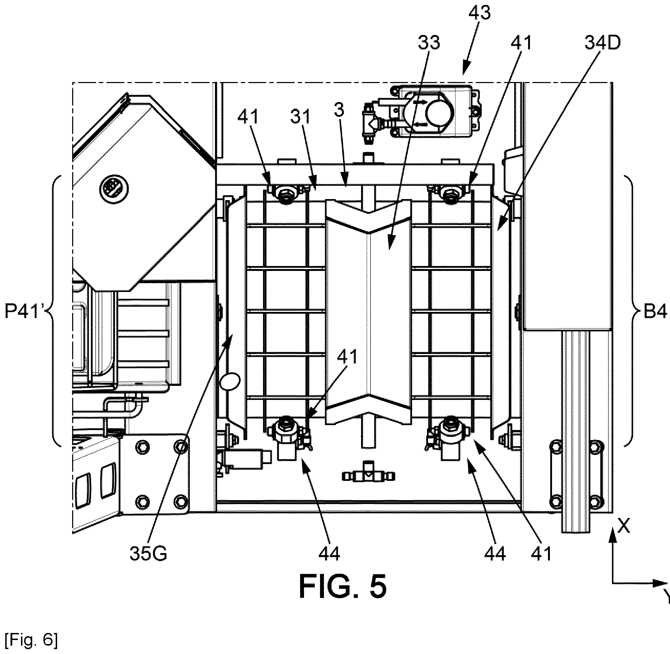

   [Fig. 5] représente une vue de dessus de la plateforme de la figure 1, dans laquelle certains éléments ont été ôtés.
**Fig. 6**

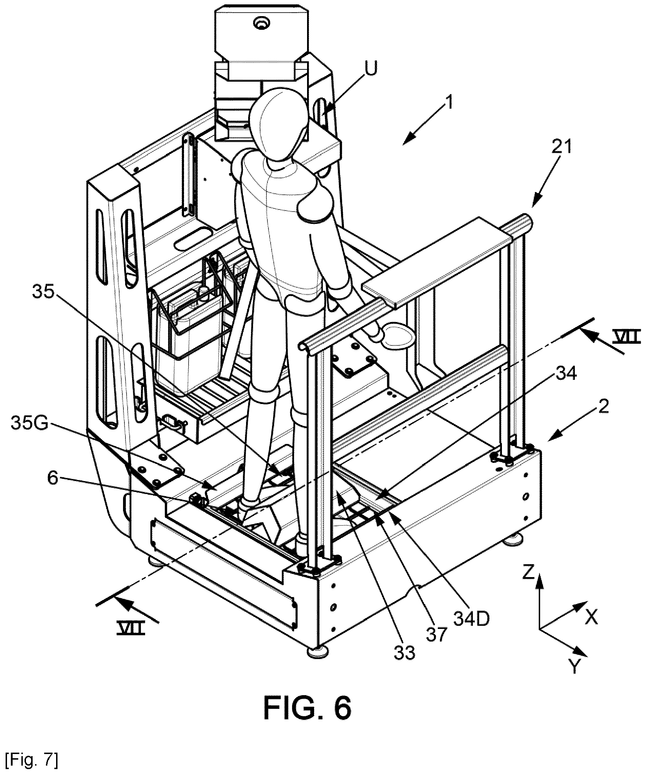

   [Fig. 6] représente une vue en perspective d'une plateforme de désinfection selon un mode de réalisation conforme à l'invention, avec un utilisateur positionné dessus.
**Fig. 7**

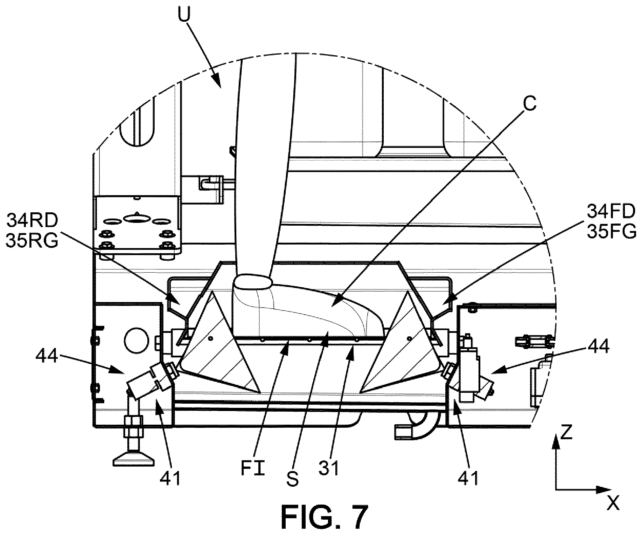

   [Fig. 7] représente une vue de détail en coupe selon la ligne VII-VII de la figure 6.

### Description des modes de réalisation

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

Dans l'ensemble de la présente demande, les directions de l'espace sont définies comme suit :
- la direction longitudinale X de la plateforme correspond à la direction d'extension des pieds d'un utilisateur lorsque celui-ci se trouve sur la plateforme en position normale d'utilisation avec la face inférieure des semelles de ses chaussures en appui sur la grille du support de semelle, comme représenté sur les exemples de réalisation des figures 1 à 7,
- la direction verticale Z de la plateforme correspond à la direction perpendiculaire au plan de la grille du support de semelle, généralement sensiblement perpendiculaire au plan du sol sur lequel la plateforme est posée en position normale d'utilisation, comme représenté sur les exemples de réalisation des figures 1 à 7,
- la direction transversale Y de la plateforme est la direction perpendiculaire à la direction longitudinale et à la direction verticale, comme représenté sur les exemples de réalisation des figures 1 à 7.

Egalement avant et arrière s'entendent selon ladite direction longitudinale X de la plateforme, avec une orientation depuis le talon des pieds de l'utilisateur vers la pointe des pieds de l'utilisateur, lorsque celui-ci se trouve sur la plateforme en position normale d'utilisation avec la face inférieure des semelles de ses chaussures en appui sur la grille du support.

De même, supérieur et inférieur s'entendent selon la direction verticale de la plateforme, avec une orientation depuis les pieds de l'utilisateur vers sa tête, lorsque celui-ci se trouve sur la plateforme en position normale d'utilisation avec la face inférieure des semelles de ses chaussures en appui sur la grille du support.

Enfin, dans l'ensemble de la présente demande, on entend par sensiblement longitudinal, transversal, vertical, axial ou radial, une orientation par rapport à la direction longitudinale, la direction transversale ou la direction verticale, avec un angle inférieur à 30°, qui peut avantageusement être nul.

De même, on entend par sensiblement parallèle, une orientation par rapport à un élément donné avec un angle inférieur à 30°, qui peut avantageusement être nul.

Enfin, dans l'ensemble de la présente demande, on entend par chaussures, les éléments chaussants portés par un utilisateur de la plateforme ou du procédé selon l'invention, qui peuvent également être des bottes, sabots ou similaires.

L'invention concerne une plateforme 1 pour la désinfection des chaussures C d'un utilisateur U par projection d'un liquide désinfectant, comportant :
- un châssis 2,
- un support 3 configuré pour recevoir en appui les chaussures C d'un utilisateur U, et solidaire du châssis 2,
- un moyen de projection 4 de liquide désinfectant, configuré pour projeter du liquide désinfectant sur les chaussures C de l'utilisateur U, et solidaire du châssis 2,
- une unité de commande 5 reliée au moyen de projection 4, et configurée de sorte à commander la projection de liquide désinfectant par le moyen de projection 4.

Selon l'invention, le support 3 comporte une grille 31 présentant une pluralité d'ouvertures 32, la grille 31 étant configurée de sorte à recevoir en appui la face inférieure FI des semelles S de chaussures C de l'utilisateur U.

Selon l'invention, le moyen de projection 4 présente une pluralité de buses 41, disposées sous la grille 31 du support 3, et configurées de sorte à projeter du liquide désinfectant sur la face inférieure FI des semelles S de chaussures C de l'utilisateur U au travers au moins une partie des ouvertures 32 de la grille 31 du support 3, sous forme de nébulisation, selon une quantité déterminée, commandée par l'unité de commande 5.

Ainsi, avec la plateforme 1 selon l'invention, le liquide désinfectant est projeté sur les chaussures C d'un utilisateur U, comme visible sur les exemples de réalisation des figures 6 et 7, par en-dessous, les chaussures C de l'utilisateur U bloquant également la propagation du liquide au-dessus d'elles.

La plateforme 1 permet ainsi une désinfection optimale des chaussures C de l'utilisateur U, et notamment de leurs semelles S, qui est la partie la plus sensible des chaussures C en ce qui concerne l'hygiène, car en contact direct avec le sol de la zone de production dans laquelle se déplace l'utilisateur U.

Les projections de liquide désinfectant au-delà du support 3, et donc du châssis 2 de la plateforme 1 sont évitées contrairement aux plateformes de l'art antérieur, ce qui évite le risque de formation de flaques sur le sol à proximité de la plateforme 1, dans lesquelles des bactéries pourraient être présentes.

Le fait d'employer une grille 31 pour le support 3 permet de garantir que la quasi-totalité du liquide désinfectant projeté par les buses 41 du moyen de projection 4 soit reçue par les chaussures C de l'utilisateur U, celui-ci pouvant aisément passer au travers des ouvertures 32 après avoir été projeté par les buses 41, tout en garantissant un maintien stable et résistant à l'utilisateur U sur la plateforme 1.

Egalement, le fait de prévoir une projection de liquide désinfectant sous forme de nébulisation permet de réduire les risques de projection de celui-ci au-delà du support 3 et donc du châssis 2 de la plateforme 1, tout en garantissant une application uniforme du liquide désinfectant sur les chaussures C de l'utilisateur U, et notamment sur la face inférieure FI des semelles S de ses chaussures C, comme visible plus particulièrement sur l'exemple de réalisation de la figure 7, ainsiqu'un temps de séchage réduit, ce qui diminue le risque que l'utilisateur U forme des flaques sur le sol à proximité de la plateforme 1, après avoir quitté ladite plateforme 1.

De plus, l'emploi d'un liquide désinfectant projeté sous forme d'une nébulisation permet de ne pas prévoir de rinçage des chaussures C après la projection du liquide désinfectant, ce qui diminue également le risque de formation de flaques à proximité de la plateforme 1, après désinfection des chaussures C d'un utilisateur U.

Enfin, cela permet également de ne pas nécessiter d'action mécanique de nettoyage supplémentaire sur les chaussures C de l'utilisateur U pour obtenir une désinfection optimale des chaussures C de l'utilisateur U, laquelle, comme expliqué ci-dessus, augmente les risques de projection de liquide désinfectant au-delà du châssis 2 de la plateforme 1, ainsi que la quantité de liquide désinfectant nécessaire au fonctionnement de la plateforme 1.

Comme visible sur l'exemple de réalisation des figures 1 et 6, le châssis 2 peut comporter une rambarde 21, configurée de sorte à permettre à l'utilisateur U de s'y tenir pour faciliter sa montée et/ou sa descente de la plateforme 1, ou encore pour lui permettre de demeurer stable pendant la projection de liquide désinfectant sur la face inférieure FI des semelles S de ses chaussures C.

La plateforme 1 peut également comporter un moyen de blocage d'accès 7, par exemple sous la forme d'un tripode monté rotatif par rapport au châssis 2, par exemple relié à l'unité de commande 5, de sorte à autoriser la traversée du support 3 par un utilisateur U. L'unité de commande 5 peut par exemple être configurée de sorte à autoriser la traversée du support 3 par un utilisateur U via la libération dudit moyen de blocage d'accès 7, uniquement après que l'utilisateur a subi un cycle de désinfection sur ladite plateforme 1.

Selon l'invention, et comme visible sur les figures 3, 4 et 7, le châssis 2 comporte en partie inférieure, sous la grille 31 du support 3 et sous les buses 41 du moyen de projection 4 un bac de récupération 22 configuré pour guider le liquide désinfectant résiduel, projeté sur les chaussures C d'un utilisateur U, mais non demeuré sur celles-ci, vers une ouverture d'évacuation 23 configurée pour être reliée à un réseau d'évacuation et de retraitement des liquides.

Selon un mode de réalisation, le moyen de projection 4 comporte un réservoir 42 de liquide désinfectant, solidaire du châssis 2, chaque buse 41 étant reliée au réservoir 42 par l'intermédiaire d'une pompe 43 et d'une électrovanne 44, intercalée entre la pompe 43 et au moins une buse 41.

Le fait de prévoir un réservoir 42 de liquide désinfectant solidaire du châssis 2 permet de ne pas avoir à relier la plateforme 1 à un réseau de distribution d'eau, comme c'est le cas avec les plateformes de l'art antérieur. Il est ainsi possible de positionner la plateforme 1 à l'endroit souhaité, et même à distance d'une arrivée d'eau du réseau de distribution d'eau.

Le volume du réservoir 42 peut être compris entre 3 L et 8 L, de préférence entre 4 L et 6 L.

Comme visible sur les exemples de réalisation des figures 1 et 6, afin de faciliter et réduire le temps de maintenance de la plateforme 1 selon l'invention, il peut être prévu deux réservoirs 42 de liquide désinfectant, avantageusement identiques, de sorte que dès qu'un des deux réservoirs 42 est vide, il suffit de raccorder l'autre à la pompe 43, avantageusement de façon automatisée par l'intermédiaire d'une électrovanne, et la plateforme 1 peut continuer de fonctionner normalement, le temps de remplacer le premier réservoir 42 vide.

Avantageusement, un moyen de verrouillage (non représenté) du réservoir 42 au châssis 2 de la plateforme 1 peut être prévu, comprenant par exemple un cadenas, de sorte à empêcher une personne non autorisée d'ôter le réservoir 42 du châssis 2.

Selon un mode de réalisation, le moyen de projection 4 comporte une unique pompe 43 configurée de sorte à alimenter simultanément l'ensemble des buses 41, et chaque buse 41 est reliée à une électrovanne 44 distincte, intercalée entre la pompe 43 et chacune des buses 41.

Cette disposition avantageuse de l'invention permet de réduire l'encombrement du moyen de projection 4, en ce qu'une seule pompe 43 est nécessaire pour assurer son fonctionnement et alimenter l'ensemble des buses 41.

De ce fait, et comme visible sur les exemples de réalisation des figures 4 et 5, la pompe 43, les buses 41 et les électrovannes 44 peuvent toutes être positionnées sous la grille 31 du support 3, ce qui réduit également l'encombrement de la plateforme 1 selon l'invention.

Le fait de prévoir une unique pompe 43 permet également de faciliter le pilotage du moyen de projection 4 par l'unité de commande 5.

La pompe 43 peut par exemple être une pompe péristaltique.

Selon un mode de réalisation, la pompe 43 est configurée de sorte à fournir un débit de liquide désinfectant compris entre 7L/heure et 20 L/heure, de préférence compris entre 10 L/heure et 15 L/heure.

Ainsi, le débit de la pompe 43 est plus faible que celui des pompes couramment employées pour les plateformes de l'état de la technique, ce qui permet de réduire la consommation de liquide désinfectant, mais également le coût de revient de la pompe 43, et donc de la plateforme 1 selon l'invention.

Afin de s'assurer que le débit d'alimentation des buses 41 est suffisant pour assurer la projection de liquide désinfectant sous forme d'une nébulisation, l'unité de commande 5 peut avantageusement être configurée de sorte à activer la pompe 43 pendant une durée prédéterminée, par exemple comprise entre 0,5 secondes et 2 secondes, de préférence environ 1 seconde, préalablement à l'ouverture des électrovannes 44.

Selon un mode de réalisation, chaque buse 41 est configurée pour projeter du liquide désinfectant sous forme d'une nébulisation de particules de diamètre compris entre 40 µm et 300 µm, de préférence entre 80 µm et 150 µm.

Avec un tel diamètre de particules de liquide désinfectant, le temps de séchage du liquide désinfectant après projection sur les chaussures C de l'utilisateur U est réduit. De plus, les particules de cette dimension peuvent aisément passer au travers des ouvertures 32 de la grille 31 du support 3, ce qui garantit une répartition sensiblement uniforme du liquide désinfectant sur les chaussures C de l'utilisateur U.

Selon un mode de réalisation, chaque buse 41 est configurée pour projeter du liquide désinfectant sous forme d'une nébulisation lorsque alimentée en liquide désinfectant avec un débit compris entre 0,05 L/min et 1,2 L/min, de préférence entre 0,07 L/min et 0,09 L/min.

Lorsque le moyen de projection 4 comporte un unique pompe 43, comme décrit ci-dessus, et fournit un débit compris entre 7L/heure et 20 L/heure, de préférence compris entre 10 L/heure et 15 L/heure, tel qu'également décrit ci-dessus, l'unité de commande 5 peut être configurée pour activer la pompe 43 et ouvrir les électrovannes 44 pendant une durée comprise entre 2 secondes et 5 secondes, de préférence entre 3 et 4 secondes, de sorte à projeter du liquide désinfectant sous forme d'une nébulisation. Une telle durée de projection permet de garantir une répartition uniforme sur l'ensemble des chaussures C d'un utilisateur U, et donc d'assurer la désinfection desdites chaussures C de l'utilisateur U, avec une consommation réduite de liquide désinfectant par rapport aux plateformes de l'état de la technique, de l'ordre d'une dizaine de mL par cycle de désinfection.

De plus, et comme le débit de la pompe 43 est bien inférieur au débit des buses 41, l'activation de la pompe 43 préalablement à l'ouverture des électrovannes 44 pendant une durée prédéterminée, par exemple comprise entre 0,5 secondes et 2 secondes, de préférence environ 1 seconde, tel que décrit ci-dessus, permet de mettre en pression le circuit d'alimentation des buses 41, de sorte que la pression y soit suffisante pour assurer la projection de liquide désinfectant sous forme d'une nébulisation et pendant ladite durée comprise entre 2 secondes et 5 secondes, de préférence entre 3 et 4 secondes, en fournissant le débit d'alimentation en liquide désinfectant souhaité aux buses 41, i.e. compris entre 0,05 L/min et 1,2 L/min, de préférence entre 0,07 L/min et 0,09 L/min.

Au-delà de cette durée, le débit fourni par la pompe 43 est insuffisant pour que les buses 41 puissent projeter du liquide désinfectant sous forme d'une nébulisation, de sorte que celui-ci est projeté desdites buses sous forme d'un jet à faible pression, lequel sous l'effet de la gravité, retombe intégralement sous la grille 31 du support, et peut avantageusement être intégralement reçu dans le bac de récupération 22 et l'ouverture d'évacuation 23 du châssis 2, et sans passer au travers des ouvertures 32 de ladite grille 31.

Ainsi, même en cas de dysfonctionnement, grâce à une telle configuration de la pompe 43 et des buses 41, après la durée déterminée de projection de produit désinfectant sous forme de nébulisation par lesdites buses 41, le liquide désinfectant ne risque pas d'être projeté au-delà du châssis 2 de la plateforme 1, ce qui supprime les risques de formation de flaques sur le sol à proximité de la plateforme 1.

Selon un mode de réalisation, le moyen de projection 4 comprend quatre buses 41 identiques, formant deux paires de buses P41, P41', avec une première paire P41 de buses 41 positionnée de sorte à projeter du liquide désinfectant sur une première chaussure C d'un utilisateur U, par exemple la chaussure droite, et une deuxième paire de buses P41' positionnées de sorte à projeter du liquide désinfectant sur une deuxième chaussure C de l'utilisateur U, par exemple la chaussure gauche.

Ainsi, chaque paire de buses P41, P41' est dédiée à la projection de liquide désinfectant sur une chaussure C de l'utilisateur U distincte, ce qui permet de diminuer la consommation de liquide désinfectant.

Selon les constations de l'inventeur, le choix de quatre buses 41 organisées en deux paires de buses P41, P41' distinctes tel que décrit ci-dessus permet d'obtenir un bon compromis entre une répartition uniforme du liquide désinfectant sur les chaussures C d'un utilisateur U et une consommation réduite de liquide désinfectant.

Selon un mode de réalisation, les buses 41 de chaque paire de buses P41, P41' sont disposées en vis-à-vis l'une de l'autre.

Comme visible sur les exemples de réalisation des figures 4, 5 et 7, une première buse 41 de chaque paire de buses P41, P41' peut être positionnée de sorte à projeter du liquide sur l'avant d'une chaussure C de l'utilisateur U, tandis que la deuxième buse 41 peut être positionnée de sorte à projeter du liquide sur l'arrière de ladite chaussure C de l'utilisateur U. Egalement, la zone de recouvrement entre la zone de projection de la première buse 41 et la zone de projection de la deuxième buse 41 de chaque paire de buses P41, P41' peut être réduite, de sorte à minimiser la consommation de liquide désinfectant.

Cette disposition avantageuse de l'invention permet d'obtenir pour chaque chaussure C d'un utilisateur U, un compromis satisfaisant entre une répartition uniforme de liquide désinfectant sur ladite chaussure C et une consommation de liquide désinfectant réduite.

Selon un mode de réalisation, les deux paires de buses P41, P41' sont symétriques entre elles, et notamment par rapport au centre de la grille 31 du support 3 selon la direction transversale Y de la plateforme 1.

Selon un mode de réalisation, le liquide désinfectant consiste en un peroxyde.

L'emploi d'un peroxyde comme liquide désinfectant permet d'éviter l'emploi d'un liquide à base d'alcool, lequel nécessite d'être mélangé à de l'eau préalablement à sa projection, ce qui évite de prévoir une alimentation en eau du moyen de projection 4, et donc de relier la plateforme 1 à une arrivée d'eau d'un réseau de distribution d'eau.

De plus, et selon les constations de l'inventeur, l'emploi d'un peroxyde comme liquide désinfectant permet d'obtenir un temps de séchage réduit de ce liquide désinfectant, une fois projeté sur les chaussures C d'un utilisateur U, typiquement inférieur à une seconde.

Selon un mode de réalisation, le peroxyde est le peroxyde d'Hydrogène.

Selon les constations de l'inventeur, le peroxyde d'Hydrogène fournit une qualité de désinfection améliorée, en neutralisant un nombre important de bactéries présentes sur les chaussures C d'un utilisateur U.

Selon un mode de réalisation, les ouvertures 32 de la grille 31 présentent, au moins pour une partie d'entre-elles, et notamment celles positionnées en regard des buses 41, selon la direction verticale Z de la plateforme 1, une forme sensiblement rectangulaire de longueur L32, selon la direction transversale Y de la plateforme 1, comprise entre 50 mm et 80 mm, de préférence comprise entre 60 mm et 75 mm, et de largeur W32, selon la direction longitudinale X de la plateforme 1, comprise entre 50 mm et 80 mm, de préférence entre 60 mm et 75 mm, deux ouvertures 32 adjacentes étant séparées entre elles d'une épaisseur de matière W31 comprise entre 3 mm et 6mm, de préférence comprise entre 4 mm et 5 mm.

Selon les constations de l'inventeur, et comme visible sur les exemples de réalisation des figures 1, 2, 5 et 6, une telle configuration des ouvertures 32 de la grille 31 permet d'obtenir un compromis optimal entre la résistance de celle-ci, de sorte à pouvoir supporter le poids d'un utilisateur U, lorsque la face inférieure FI de la semelle S de ses chaussures C se trouve en appui sur celle-ci, et la distribution de liquide désinfectant projeté par les buses 41 du moyen de projection 4 sur l'ensemble de la surface des chaussures C de l'utilisateur U, la surface des ouvertures 32 étant suffisamment grande pour laisser passer suffisamment de liquide désinfectant.

Avantageusement, les ouvertures 32 peuvent être de forme carrée.

Alternativement, et dans sortir du cadre de la présente invention, les ouvertures 32 pourraient présenter une forme différente, par exemple circulaire ou polygonale, avec une surface équivalente à celle obtenue avec les dimensions décrites ci-dessus, et une épaisseur de matière W31 les séparant identique.

Selon un mode de réalisation, la grille 31 comporte une pluralité de fils métalliques F31 soudés entre eux, formant lesdites ouvertures 32, le diamètre des fils étant compris entre 3 mm et 6mm, de préférence compris entre 4 mm et 5 mm.

Une telle grille 31 est ainsi simple à réaliser et de coût de revient réduit, et notamment lorsque celle-ci présente une forme complexe.

La grille 31 peut être réalisée en une seule partie, ou, comme visible sur l'exemple de réalisation de la figure 3, en plusieurs parties indépendantes, ici deux, et notamment pour réduire son coût de revient et/ou pour simplifier sa fabrication.

La section des fils métalliques F31 peut être circulaire, ou polygonale.

Les fils F31 peuvent être en acier inoxydable.

La grille 31, peut avantageusement être configurée de sorte à ne présenter aucun corps creux, de sorte à faciliter son nettoyage, et diminuer le risque de prolifération de bactéries, inaccessibles depuis l'intérieur des corps creux.

Le châssis 2 de la plateforme 1, peut également être configuré de sorte à ne présenter aucun corps creux, et notamment à proximité de ladite grille 31, et de sorte à aussi faciliter son nettoyage, et diminuer le risque de prolifération de bactéries.

Selon un mode de réalisation, le support 3 comporte un élément de séparation 33 des pieds d'un utilisateur, fixé sur le support 3, de préférence sensiblement au milieu de la grille 31, selon la direction transversale Y de la plateforme 1, et configuré de sorte délimiter sur le support 3, selon la direction transversale Y de la plateforme 1, un premier espace 34 configuré pour recevoir la chaussure C droite d'un utilisateur U et un deuxième espace 35 configuré pour recevoir la chaussure C gauche d'un utilisateur U.

Dans un tel mode de réalisation, comme visible sur l'exemple de réalisation de la figure 3, la grille 31 peut être réalisée en deux parties : une première partie appartenant audit premier espace 34 et la deuxième partie appartenant audit deuxième espace 35.

Dans un tel mode de réalisation, les buses 41 du moyen de projection 4 peuvent être positionnées, pour une partie, par exemple pour une première paire de buse P41 telle que décrite ci-dessus, en vis-à-vis, selon l'axe vertical Z de la plateforme 1, du premier espace 34, et pour une autre partie, par exemple pour la deuxième paire de buse P41' telle que décrite ci-dessus, en vis-à-vis, selon l'axe vertical Z de la plateforme 1 du deuxième espace 35.

L'emploi d'un élément de séparation 33, comme visible sur les exemples de réalisation des figures 6 et 7, permet ainsi de guider l'utilisateur U pour que celui-ci positionne de façon optimale ses chaussures C sur la grille 31 du support 3, de sorte à ce que celles-ci reçoivent du liquide désinfectant.

L'élément de séparation 31 peut ainsi avantageusement être configuré de sorte à recevoir en appui chacune des chaussures C d'un utilisateur U.

Selon un mode de réalisation, le support 3 comporte :
- une paroi de butée droite 34D, configurée pour délimiter en combinaison avec l'élément de séparation 33 le premier espace 34 du support 3, selon la direction transversale Y de la plateforme 1, et/ou
- une paroi de butée avant droite 34FD et une paroi de butée arrière droite 34RD, configurées pour délimiter le premier espace 34 du support 3, selon la direction longitudinale X de la plateforme 1, respectivement à l'avant et à l'arrière, et/ou
- une paroi de butée gauche 35G, configurée pour délimiter en combinaison avec l'élément de séparation 33 le deuxième espace 35 du support 3, selon la direction transversale Y de la plateforme 1, et/ou
- une paroi de butée avant gauche 35FG et une paroi de butée arrière gauche 35RG, configurées pour délimiter le deuxième espace 35 du support 3, selon la direction longitudinale X de la plateforme 1, respectivement à l'avant et à l'arrière.

Les pieds d'un utilisateur U sont ainsi guidés de façon optimale, de sorte que ses chaussures C, respectivement droite et gauche, se trouvent positionnées pour recevoir du liquide désinfectant, ce qui permet également d'éviter la projection de liquide désinfectant au-delà du support 3 et donc du châssis 2 de la plateforme 1.

Notamment, les pieds de l'utilisateur U peuvent ainsi être positionnés de sorte que la première paire de buses P41, telle que décrite ci-dessus, se trouve en vis-à-vis de la chaussure C droite de l'utilisateur U, selon la direction verticale Z de la plateforme 1, et que la deuxième paire de buses P41', telle que décrite ci-dessus, se trouve en vis-à-vis de la chaussure C gauche de l'utilisateur U, selon la direction verticale Z de la plateforme 1.

Comme visible sur les exemples de réalisation des figures 1 à 4, la paroi de butée avant droite 34FD, la paroi de butée arrière droite 34RD, la paroi de butée avant gauche 35DG et la paroi de butée arrière gauche 35RG peuvent s'étendre sensiblement selon la direction transversale Y de la plateforme 1, et la paroi de butée droite 35D, et la paroi de butée gauche 35G peuvent s'étendre sensiblement selon la direction longitudinale X de la plateforme 1.

Egalement la paroi de butée avant droite 34FD et la paroi de butée avant gauche 35FG peuvent être ménagées d'un seul tenant et venues de matière. De même, la paroi de butée arrière droite 34RD et la paroi debutée arrière gauche 35RG peuvent être ménagées d'un seul tenant et venues de matière, et afin de faciliter la fabrication du support 3 de la plateforme 1 selon l'invention.

Egalement, comme visible sur l'exemple de réalisation de la figure 4, la paroi de butée avant droite 34FD et/ou la paroi de butée arrière droite 34RD et/ou la paroi de butée avant gauche 35FG et/ou la paroi de butée arrière gauche 35RG peuvent être conformées de sorte à guider le liquide désinfectant projeté par les buses 41 vers les chaussures C de l'utilisateur U, lorsque la face inférieure FI des semelles S de chaussures C de l'utilisateur U est reçue en appui sur la grille 31 du support 3, et à éviter les projections de liquide désinfectant au-delà du support 3 et donc du châssis 2 de la plateforme 1.

A cet effet, la paroi de butée avant droite 34FD et/ou la paroi de butée arrière droite 34RD et/ou la paroi de butée avant gauche 35FG et/ou la paroi de butée arrière gauche 35RG peuvent comporter une ou plusieurs faces inclinées par rapport à la direction verticale Z de la plateforme 1, autour de la direction transversale Y de la plateforme 1, de sorte à agir à la manière de déflecteurs.

De même, comme visible sur l'exemple de réalisation de la figure 3, la paroi de butée droite 34D et/ou la paroi de butée gauche 35G peuvent également être conformées de sorte à guider le liquide désinfectant projeté par les buses 41 vers les chaussures C de l'utilisateur U, lorsque la face inférieure FI des semelles S de chaussures C de l'utilisateur U est reçue en appui sur la grille 31 du support 3, et à éviter les projections de liquide désinfectant au-delà du support 3 et donc du châssis 2 de la plateforme 1.

A cet effet, la paroi de butée droite 34D et/ou la paroi de butée avant gauche 35FG et/ou la paroi de butée gauche 35G peuvent comporter une ou plusieurs faces inclinées par rapport à la direction verticale Z de la plateforme 1, autour de la direction longitudinale X de la plateforme 1, de sorte à agir à la manière de déflecteurs.

Selon un mode de réalisation, une première partie des buses 41, et notamment la première paire de buses P41 telle que décrite ci-dessus, sont configurées de sorte à projeter du liquide désinfectant uniquement dans le premier espace 34 du support 3 et une deuxième partie des buses 41, et notamment la deuxième paire de buses P41' telle que décrite ci-dessus, sont configurées de sorte à projeter du liquide désinfectant uniquement dans le deuxième espace 35 du support 3.

Cette disposition avantageuse de l'invention permet de réduire la consommation de liquide désinfectant, en ce qu'il n'y a pas de zone de recouvrement entre la zone de projection de liquide désinfectant de la première partie des buses 41 et la zone de projection de liquide désinfectant de la deuxième partie des buses 41.

A cet effet, et comme visible plus particulièrement sur l'exemple de réalisation de la figure 3, la première partie des buses 41 peut être positionnée en vis-à-vis du premier espace 34, selon la direction verticale Z de la plateforme 1, tandis que la deuxième partie des buses 41 peut être positionnée en vis-à-vis du deuxième espace 35, selon la direction verticale Z de la plateforme 1.

En alternative ou en complément, comme visible plus particulièrement sur l'exemple de réalisation de la figure 3, l'élément de séparation 33 du support peut également être configuré de sorte à empêcher la projection de liquide désinfectant émis par la première partie des buses 41 vers le deuxième espace 35, et/ou pour empêcher la projection de liquide désinfectant émis par la première partie des buses 41 vers le premier espace 34.

Selon un mode de réalisation, l'élément de séparation 33 et/ou la paroi de butée droite 34D et/ou la paroi de butée avant droite 34FD et/ou la paroi de butée arrière droite 34RD et/ou la paroi de butée gauche 35G et/ou la paroi de butée avant gauche 35FG et/ou la paroi de butée arrière gauche 35RG sont configurés de sorte à empêcher le risque de blessure de l'utilisateur U.

A cet effet, elles peuvent être fabriquées de sorte à présenter un minimum d'arêtes vives, c'est-à-dire que toutes ou la plupart des arêtes entre deux faces successives sont formées arrondies.

Selon un mode de réalisation, la plateforme 1 comprend en outre un moyen de détection 6 des chaussures C d'un utilisateur U, relié à l'unité de commande 5, configuré de sorte à détecter la présence des chaussures C d'un utilisateur U sur la grille 31 du support 3, l'unité de commande 5 étant configurée pour commander la projection de liquide désinfectant sur les chaussures C d'un utilisateur U par le moyen de projection 4 après la détection de la présence des deux chaussures C de l'utilisateur U sur la grille 31 du support 3.

Cette disposition avantageuse de l'invention limite la consommation de produit désinfectant, en ce que celui-ci n'est projeté via le moyen de projection 4, que si un utilisateur U se trouve sur la plateforme avec ses deux chaussures C sur la grille 31 du support 3. Cela permet d'éviter que du liquide désinfectant soit projeté via le moyen de projection 4 alors qu'aucun n'utilisateur U ne se trouve sur la plateforme U ou que ses chaussures C ne se trouvent pas sur la grille 31 du support 3.

Grâce à cette disposition avantageuse, la désinfection des chaussures C d'un utilisateur U peut également être entièrement automatisée, ce qui facilite l'utilisation de la plateforme 1 selon l'invention. En effet, l'unité de commande 5 peut être configurée pour commander automatiquement la projection de liquide désinfectant sur les chaussure C d'un utilisateur U par le moyen de projection 4 après la détection de la présence des deux pieds de l'utilisateur U sur la grille 31 du support 3, et sans requérir aucune action de l'utilisateur U ou d'une tierce personne pour commander l'activation du moyen de projection 4.

Selon un mode de réalisation, le moyen de détection 6 est configuré pour détecter la présence de la chaussure C droite d'un utilisateur U dans le premier espace 34 du support 3, tel que décrit ci-dessus, et pour détecter la présence de la chaussure C gauche de l'utilisateur U dans le deuxième espace 35 du support 3, tel que décrit ci-dessus.

L'unité de commande 5 peut alors être configurée pour commander la projection de liquide désinfectant sur les chaussures C de l'utilisateur U par le moyen de projection 4 après la détection de la présence de la chaussure C droite d'un utilisateur U dans le premier espace 34 du support 3 et de la présence de la chaussure C gauche de l'utilisateur U dans le deuxième espace 35 du support 3.

Grâce à cette disposition avantageuse de l'invention, le moyen de détection 6 permet, en plus de s'assurer qu'un utilisateur U ait bien ses chaussures C sur la grille 31 du support 3 de la plateforme 1, de s'assurer que l'utilisateur U a positionné correctement ses chaussures C sur la grille 31 du support 3, de sorte que du liquide désinfectant soit projeté sur celles-ci par le moyen de projection 4, de sorte à obtenir une désinfection optimale desdites chaussures C.

Selon un mode de réalisation, le moyen de détection 6 comporte un moyen d'émission 61 d'un signal, par exemple un signal lumineux, de préférence infrarouge, et un moyen de réception 62 du signal émis par le moyen d'émission 61.

Un tel moyen de détection 6 présente une fiabilité élevée de détection, même dans un milieu perturbé, comme un site de production industrielle, pour un faible coût de revient.

Un tel moyen de détection 6 n'est notamment pas sensible à la présence de liquide, et notamment de liquide désinfectant, qui ne perturbe pas sa fiabilité de détection.

Comme visible sur l'exemple de réalisation de la figure 3, le moyen de détection 6 peut comporter un premier moyen d'émission 61 coopérant avec un premier de réception 62 configurés de sorte à détecter la présence de la chaussure C droite d'un utilisateur U dans le premier espace 34 du support 3 et un deuxième moyen d'émission 61 coopérant avec un deuxième moyen de réception 62 configurés de sorte à détecter la présence de la chaussure C gauche de l'utilisateur U dans le deuxième espace 35 du support 3.

Le premier moyen d'émission 61 peut être solidaire du châssis 2 et positionné à proximité de la buté droite 34D, décalé selon la direction transversale Y de la plateforme 1 du premier espace 34, tandis que le deuxième moyen d'émission 61 peut également être solidaire du châssis 2 et positionné à proximité de la butée gauche 35G, décalé selon la direction transversale Y de la plateforme 1 du deuxième espace 35. La butée droite 34D, respectivement la butée gauche 35G peuvent alors comporter un perçage traversant configuré de sorte à laisser passer le signal émis par le premier moyen d'émission 61, respectivement le deuxième moyen d'émission 61, au travers de la butée droite 34D, respectivement de la butée gauche 35G.

Le premier moyen de réception 62 et/ou le deuxième moyen de réception 62 peuvent être logés dans l'élément de séparation 33. L'élément de séparation 33 peut alors comporter un perçage traversant configuré de sorte à laisser passer le signal émis par le premier moyen d'émission 61 et/ou un perçage traversant configuré de sorte à laisser passer le signal émis par le deuxième moyen d'émission 61.

Alternativement, le moyen d'émission 61 et le moyen de réception 62 du moyen de détection 6 peuvent être positionnés côte à côte, ou encore être confondus en un unique moyen de détection et réception 61, 62, et de sorte à minimiser l'encombrement du moyen de détection 6.

L'invention concerne également un procédé de désinfection des chaussures C d'un utilisateur U mis en oeuvre avec une plateforme 1 selon l'un des modes de réalisation décrits précédemment, comprenant l'étape :
- lancement d'un cycle de désinfection avec projection de liquide désinfectant sous forme de nébulisation sur les chaussures C d'un utilisateur U, par les buses 41 du moyen de projection 4 au travers des ouvertures 32 de la grille 31 du support 3, sur laquelle est reçue en appui la face inférieure FI des semelles S de chaussures C de l'utilisateur U.

L'ensemble des dispositions décrites précédemment concernant le fonctionnement de la plateforme 1 selon l'invention pour la désinfection des chaussures C d'un utilisateur U par projection de liquide désinfectant sous forme de nébulisation via les buses 41 du moyen de projection 4 sur lesdites chaussures C de l'utilisateur U, s'appliquent au procédé selon l'invention.

Notamment, un tel procédé de désinfection permet d'assurer une désinfection optimale des chaussures C d'un utilisateur U, en un temps réduit, et en réduisant le risque de projection de liquide désinfectant au-delà du châssis 2 de la plateforme 1.

Un tel procédé de désinfection permet également un temps de séchage réduit du liquide désinfectant sur les chaussures C de l'utilisateur U, ce qui évite la formation de flaques de liquide désinfectant sur le sol à proximité de la plateforme 1.

Selon un mode de réalisation du procédé, la plateforme 1 comprend un moyen de détection 6 des chaussures C d'un utilisateur U, relié à l'unité de commande 5, configuré de sorte à détecter la présence des chaussures C d'un utilisateur U sur la grille 31 du support 3, et l'unité de commande 5 est configurée pour lancer un cycle de désinfection des chaussures C d'un utilisateur U avec projection de liquide désinfectant par le moyen de projection 4 après la détection de la présence des deux pieds de l'utilisateur U sur la grille 31 du support 3.

Selon un tel mode de réalisation, ledit procédé comprend en outre, préalablement à l'étape de lancement du cycle de désinfection, une étape de détection de la présence des pieds d'un utilisateur U sur la grille 31 du support 3, l'étape de lancement du cycle de désinfection n'étant exécutée que si la présence des chaussures C de l'utilisateur U sur la grille 31 du support 3 est détectée.

L'ensemble des dispositions décrites précédemment concernant le fonctionnement de la plateforme 1 lorsque celle-ci comporte un tel moyen de détection 6, tel que décrit ci-dessus, s'appliquent au procédé selon un tel mode de réalisation.

Selon un mode de réalisation du procédé, le moyen de projection 4 de la plateforme 1 comporte une unique pompe 43 configurée de sorte à alimenter simultanément l'ensemble des buses 41, et chaque buse 41 est reliée à une électrovanne 44 distincte, intercalée entre la pompe 43 et chacune des buses 41, et la pompe 43 est configurée de sorte à fournir un débit de liquide désinfectant compris entre 7L/heure et 20 L/heure, de préférence compris entre 10 L/heure et 15 L/heure, et chaque buse 41 est configurée pour projeter du liquide désinfectant sous forme d'une nébulisation lorsque alimentée en liquide désinfectant avec un débit compris entre 0,05 L/min et 1,2 L/min, de préférence entre 0,07 L/min et 0,09 L/m i n.

Selon un tel mode de réalisation, l'étape de lancement du cycle de désinfection comporte les sous étapes successives :
- activation de la pompe 43 pendant une durée comprise entre 0,5 secondes et 2 secondes, de préférence environ 1 seconde, les électrovannes 44 demeurant fermées,
- ouverture des électrovannes 44 et maintien de la pompe 43 activée pendant une durée comprise entre 2 secondes et 5 secondes, de préférence entre 3 et 4 secondes, de sorte à projeter du liquide désinfectant au travers des ouvertures 32 de la grille 31 du support 3 par l'intermédiaire des buses 41.

Comme expliqué ci-dessus, cette disposition avantageuse du procédé permet de s'assurer que le débit d'alimentation des buses 41 est suffisant pour assurer la projection de liquide désinfectant.

Cela permet également permet de garantir une répartition uniforme sur les chaussures C d'un utilisateur U, et donc d'assurer la désinfection desdites chaussures C de l'utilisateur U, avec une consommation réduite de liquide désinfectant par rapport aux plateformes de l'état de la technique, de l'ordre d'une dizaine de mL par cycle de désinfection.

Enfin, et comme le débit de la pompe 43 est bien inférieur au débit des buses 41, l'activation de la pompe 43 préalablement à l'ouverture des électrovannes 44 pendant une durée prédéterminée, par exemple comprise entre 0,5 secondes et 2 secondes, de préférence environ 1 seconde, permet de mettre en pression le circuit d'alimentation des buses 41, de sorte que la pression y soit suffisante pour assurer la projection de liquide désinfectant sous forme d'une nébulisation et pendant ladite durée comprise entre 2 secondes et 5 secondes, de préférence entre 3 et 4 secondes, en fournissant le débit d'alimentation en liquide désinfectant souhaité aux buses 41, i.e. compris entre 0,05 L/min et 1,2 L/min, de préférence entre 0,07 L/min et 0,09 L/min.

Au-delà de cette durée, le débit fourni par la pompe 43 est insuffisant pour que les buses 41 puissent projeter du liquide désinfectant sous forme d'une nébulisation, de sorte que celui-ci est projeté desdites buses sous forme d'un jet à faible pression, lequel sous l'effet de la gravité, retombe intégralement sous la grille 31 du support 3, et est éventuellement intégralement reçu dans le bac de récupération 22 et l'ouverture d'évacuation 23 du châssis 2, et sans passer au travers des ouvertures 32 de ladite grille 31.

Ainsi, même en cas de dysfonctionnement, grâce à une telle configuration de la pompe 43 et des buses 41, après la durée déterminée de projection de produit désinfectant sous forme de nébulisation par lesdites buses 41, le liquide désinfectant ne risque pas d'être projeté au-delà du châssis 2 de la plateforme 1, ce qui supprime les risques de formation de flaques sur le sol à proximité de la plateforme 1.

Naturellement, d'autres modes de réalisation auraient pu être envisagés par l'Homme du métier sans pour autant sortir du cadre de l'invention définie par les revendications ci-après.

### Liste des signes de référence

1. Plateforme de désinfection
X. Direction longitudinale
Y. Direction transversale
Z. Direction verticale
2. Châssis
21. Rambarde
22. Bac de récupération
23. Ouverture d'évacuation
3. Support
31. Grille
F31. Fil métallique
W31. Epaisseur de matière
32. Ouverture
L32. Longueur
W32. Largeur
33. Elément de séparation
34. Premier espace
35. Deuxième espace
34D. Butée droite
34FD. Butée avant droite
34RD. Butée arrière droite
35G. Butée gauche
35FG. Butée avant gauche
35RG. Butée arrière gauche
U. Utilisateur
FI. Face inférieure
S. Semelles
C. Chaussures
4. Moyen de projection
41. Buses
P41, P41'. Paire de buses
42. Réservoir
43. Pompe
44. Electrovanne
5. Unité de commande
6. Moyen de détection
61. Moyen d'émission
62. Moyen de réception

## Revendications

1. Plateforme (1) pour la désinfection des chaussures (C) d'un utilisateur (U) par projection d'un liquide désinfectant, comportant :
- un châssis (2),
- un support (3) configuré pour recevoir les chaussures (C) d'un utilisateur (U), et solidaire du châssis (2),
- un moyen de projection (4) de liquide désinfectant, configuré pour projeter du liquide désinfectant sur les chaussures (C) de l'utilisateur (U), et solidaire du châssis (2),
- une unité de commande (5) reliée au moyen de projection (4), et configurée de sorte à commander la projection de liquide désinfectant par le moyen de projection (4),
dans lequel le support (3) comporte une grille (31) présentant une pluralité d'ouvertures (32), la grille (31) étant configurée de sorte à recevoir en appui la face inférieure (FI) des semelles (S) de chaussures (C) de l'utilisateur (U),
dans lequel le moyen de projection (4) présente une pluralité de buses (41), disposées sous la grille (31) du support (3), et configurées de sorte à projeter du liquide désinfectant sur les chaussures (C) de l'utilisateur (U) au travers au moins une partie des ouvertures (32) de la grille (31) du support (3), sous forme de nébulisation, selon une quantité déterminée, commandée par l'unité de commande (5),
dans laquelle le châssis (2) comporte en partie inférieure, sous la grille (31) du support (3) et sous les buses (41), un bac de récupération (22),
dans laquelle le moyen de projection (4) comporte un réservoir (42) de liquide désinfectant, solidaire du châssis (2), et
dans laquelle le moyen de projection (4) comporte une unique pompe (43) configurée de sorte à alimenter simultanément l'ensemble des buses (41), **caractérisé en ce que** chaque buse (41) est reliée à une électrovanne (44) distincte, intercalée entre la pompe (43) et chacune des buses (41),
dans laquelle ladite unité de commande est configurée de sorte à commander la projection de liquide désinfectant par le moyen de projection (4), par le lancement d'un cycle de désinfection avec projection de liquide désinfectant sous forme de nébulisation sur les chaussures (C) d'un utilisateur (U), par les buses (41) du moyen de projection (4) au travers des ouvertures (32) de la grille (31) du support (3), sur lequel est reçue en appui la face inférieure (FI) des semelles (S) de chaussures (C) de l'utilisateur (U) et dans laquelle l'étape de lancement du cycle de désinfection comportant les sous étapes successives :
- activation préalable de la pompe (43) avec les électrovannes (44) demeurant fermées pendant une durée prédéterminée comprise entre 0,5 secondes et 2 secondes, de préférence environ 1 seconde,
- ouverture des électrovannes (44) et maintien de la pompe (43) activée pendant une durée comprise entre 2 secondes et 5 secondes, de préférence entre 3 et 4 secondes, de sorte à projeter du liquide désinfectant au travers des ouvertures (32) de la grille (31) du support (3) par l'intermédiaire des buses (41),
et dans laquelle le débit de la pompe (43) est bien inférieur au débit des buses (41), et dans laquelle l'activation de la pompe (43) préalablement à l'ouverture des électrovannes (44) pendant la durée prédéterminée, comprise entre 0,5 secondes et 2 secondes, de préférence environ 1 seconde, est configurée pour mettre en pression le circuit d'alimentation des buses (41), de sorte que la pression y soit suffisante pour assurer la projection de liquide désinfectant sous forme d'une nébulisation et pendant ladite durée d'activation de la pompe comprise entre 2 secondes et 5 secondes, de préférence entre 3 et 4 secondes, en fournissant le débit d'alimentation en liquide désinfectant souhaité aux buses (41), par exemple compris entre 0,05 L/min et 1,2 L/min, de préférence entre 0,07 L/min et 0,09 L/min, et dans laquelle en cas de dysfonctionnement, au-delà de cette durée d'activation de la pompe comprise entre 2s et 4s, le débit fourni par la pompe (43) est insuffisant pour que les buses 41 puissent projeter du liquide désinfectant sous forme d'une nébulisation, de sorte que celui-ci est projeté desdites buses sous forme d'un jet à faible pression, lequel sous l'effet de la gravité, retombe intégralement sous la grille (31) du support (3), et est éventuellement intégralement reçu dans le bac de récupération (22) et l'ouverture d'évacuation (23) du châssis (2), et sans passer au travers des ouvertures (32) de ladite grille (31).

2. Plateforme (1) selon la revendication 1, dans laquelle la pompe (43) est configurée de sorte à fournir un débit de liquide désinfectant compris entre 7L/heure et 20 L/heure, de préférence compris entre 10 L/heure et 15 L/heure.

3. Plateforme (1) selon l'une des revendications 1 à 2, dans laquelle chaque buse (41) est configurée pour projeter du liquide désinfectant sous forme d'une nébulisation de particules de diamètre compris entre 40 µm et 300 µm, de préférence entre 80 µm et 150 µm.

4. Plateforme (1) selon l'une des revendications 1 à 3, dans laquelle chaque buse (41) est configurée pour projeter du liquide désinfectant sous forme d'une nébulisation lorsque alimentée en liquide désinfectant avec un débit compris entre 0,05 L/min et 1,2 L/min, de préférence entre 0,07 L/min et 0,09 L/min.

5. Plateforme (1) selon l'une des revendications 1 à 4, dans laquelle le moyen de projection (4) comprend quatre buses (41) identiques, formant deux paires de buses (P41, P41'), avec une première paire de buses (P41) positionnée de sorte à projeter du liquide désinfectant sur la face inférieure (FI) de la semelle (S) d'une première chaussure (C) d'un utilisateur (U), et une deuxième paire de buses (P41') positionnée de sorte à projeter du liquide désinfectant sur la face inférieure (FI) de la semelle (S) d'une deuxième chaussure (C) de l'utilisateur (U) et dans laquelle en particulier les buses (41) de chaque paire de buses (P41, P41') sont disposées en vis-à-vis l'une de l'autre, et/ou les deux paires de buses (P41, P41') sont symétriques entre elles.

6. Plateforme (1) selon l'une des revendications 1 à 5, dans laquelle le liquide désinfectant consiste en un peroxyde, et en particulier le peroxyde est le peroxyde d'Hydrogène.

7. Plateforme (1) selon l'une des revendications 1 à 6, dans laquelle les ouvertures (32) de la grille (31) présentent au moins pour une partie d'entre-elles une forme sensiblement rectangulaire de longueur (L32), selon la direction transversale (Y) de la plateforme (1), comprise entre 50 mm et 80 mm, de préférence comprise entre 60 mm et 75 mm, et de largeur (W32), selon la direction longitudinale (X) de la plateforme (1), comprise entre 50 mm et 80 mm, de préférence entre 60 mm et 75 mm, deux ouvertures (32) adjacentes étant séparées entre elles d'une épaisseur de matière (W31) comprise entre 3 mm et 6mm, de préférence comprise entre 4 mm et 5 mm et en particulier la grille (31) comporte une pluralité de fils métalliques (F31) soudés entre eux, formant lesdites ouvertures (32), le diamètre des fils étant compris entre 3 mm et 6mm, de préférence compris entre 4 mm et 5 mm.

8. Plateforme (1) selon l'une des revendications 1 à 7, dans laquelle le support (3) comporte un élément de séparation (33) des pieds d'un utilisateur, fixé sur le support (3), de préférence sensiblement au milieu de la grille (31), selon la direction transversale (Y) de la plateforme (1), et configuré de sorte à délimiter sur le support (3), selon la direction transversale (Y) de la plateforme (1), un premier espace (34) configuré pour recevoir la chaussure (C) droite d'un utilisateur (U) et un deuxième espace (35) configuré pour recevoir la chaussure (C) gauche d'un utilisateur (U) et en particulier le support (3) comporte :
- une paroi de butée droite (34D), configurée pour délimiter en combinaison avec l'élément de séparation (33) le premier espace (34) du support (3), selon la direction transversale (Y) de la plateforme (1), et/ou
- une paroi de butée avant droite (34FD) et une paroi de butée arrière droite (34RD), configurées pour délimiter le premier espace (34) du support (3), selon la direction longitudinale (X) de la plateforme (1), respectivement à l'avant et à l'arrière, et/ou
- une paroi de butée gauche (35G), configurée pour délimiter en combinaison avec l'élément de séparation (33) le deuxième espace (35) du support (3), selon la direction transversale (Y) de la plateforme (1), et/ou
- une paroi de butée avant gauche (35FG) et une paroi de butée arrière gauche (35RG), configurées pour délimiter le deuxième espace (35) du support (3), selon la direction longitudinale (X) de la plateforme (1), respectivement à l'avant et à l'arrière.

9. Plateforme selon la revendication la revendication 8, dans laquelle une première partie des buses (41) sont configurées de sorte à projeter du liquide désinfectant uniquement dans le premier espace (34) du support (3) et une deuxième partie des buses (41) sont configurées de sorte à projeter du liquide désinfectant uniquement dans le deuxième espace (35) du support (3).

10. Plateforme (1) selon l'une des revendications 1 à 9, comprenant en outre un moyen de détection (6) des chaussures (C) d'un utilisateur (U), relié à l'unité de commande (5), configuré de sorte à détecter la présence des chaussures (C) d'un utilisateur (U) sur la grille (31) du support (3), l'unité de commande (5) étant configurée pour commander la projection de liquide désinfectant sur les chaussures (C) d'un utilisateur (U) par le moyen de projection (4) après la détection de la présence des deux chaussures (C) de l'utilisateur (U) sur la grille (31) du support (3).

11. Plateforme (1) selon la revendication 10 prise en combinaison avec la revendication 9 dans laquelle le moyen de détection (6) est configuré pour détecter la présence de la chaussure (C) droite d'un utilisateur (U) dans le premier espace (34) du support (3) et pour détecter la présence de la chaussure (C) gauche de l'utilisateur (U) dans le deuxième espace (35) du support (3), et dans laquelle l'unité de commande (5) est configurée pour commander la projection de liquide désinfectant sur les chaussures (C) d'un utilisateur (U) par le moyen de projection (4) après la détection de la présence de la chaussure (C) droite de l'utilisateur (U) dans le premier espace (34) du support (3) et de la présence de la chaussure (C) gauche de l'utilisateur (U) dans le deuxième espace (35) du support (3).

12. Plateforme (1) selon la revendication 10 ou 11, dans laquelle le moyen de détection (6) comporte un moyen d'émission (61) d'un signal, par exemple un signal lumineux, de préférence infrarouge, et un moyen de réception (62) du signal émis par le moyen d'émission (61).

13. Plateforme (1) selon la revendication 12 prise en combinaison avec la revendication 11, dans laquelle le moyen de détection (6) comporte :
- un premier moyen d'émission (61), configuré pour émettre un signal dans le premier espace (34) du support (3), et un premier moyen de réception (62), configuré pour recevoir le signal émis par le premier moyen d'émission (61), et
- un deuxième moyen d'émission (61), configuré pour émettre un signal dans le deuxième espace (35) du support (3), et un deuxième moyen de réception (62), configuré pour recevoir le signal émis par le deuxième moyen d'émission (61).

14. Procédé de désinfection des chaussures (C) d'un utilisateur (U) mis en oeuvre avec une plateforme (1) selon l'une des revendications 1 à 13, comprenant l'étape :
- lancement d'un cycle de désinfection avec projection de liquide désinfectant sous forme de nébulisation sur les chaussures (C) d'un utilisateur (U), par les buses (41) du moyen de projection (4) au travers des ouvertures (32) de la grille (31) du support (3), sur lequel est reçue en appui la face inférieure (FI) des semelles (S) de chaussures (C) de l'utilisateur (U).

15. Procédé de désinfection selon la revendication 14 prise en combinaison avec l'une des revendications 10 à 13, comprenant en outre, préalablement à l'étape de lancement du cycle de désinfection, une étape de détection de la présence des chaussures (C) d'un utilisateur (U) sur la grille (31) du support (3), l'étape de lancement du cycle de désinfection n'étant exécutée que si la présence des chaussures (C) de l'utilisateur (U) sur la grille (31) du support (3) est détectée.

16. Procédé de désinfection selon la revendication 14 ou 15, prise en combinaison avec les revendications 1 et 3, dans lequel, l'étape de lancement du cycle de désinfection comporte les sous étapes successives :
- activation préalable de la pompe (43) avec les électrovannes (44) demeurant fermées pendant une durée prédéterminée comprise entre 0,5 secondes et 2 secondes, de préférence environ 1 seconde,
- ouverture des électrovannes (44) et maintien de la pompe (43) activée pendant une durée comprise entre 2 secondes et 5 secondes, de préférence entre 3 et 4 secondes, de sorte à projeter du liquide désinfectant au travers des ouvertures (32) de la grille (31) du support (3) par l'intermédiaire des buses (41) et dans lequel le débit de la pompe (43) est bien inférieur au débit des buses (41), et dans lequel l'activation de la pompe (43) préalablement à l'ouverture des électrovannes (44) pendant la durée prédéterminée, comprise entre 0,5 secondes et 2 secondes, de préférence environ 1 seconde, est configurée pour mettre en pression le circuit d'alimentation des buses (41), de sorte que la pression y soit suffisante pour assurer la projection de liquide désinfectant sous forme d'une nébulisation et pendant ladite durée d'activation de la pompe comprise entre 2 secondes et 5 secondes, de préférence entre 3 et 4 secondes, en fournissant le débit d'alimentation en liquide désinfectant souhaité aux buses (41), par exemple compris entre 0,05 L/min et 1,2 L/min, de préférence entre 0,07 L/min et 0,09 L/min, et dans laquelle en cas de dysfonctionnement, au-delà de cette durée d'activation de la pompe comprise entre 2s et 4s, le débit fourni par la pompe (43) est insuffisant pour que les buses 41 puissent projeter du liquide désinfectant sous forme d'une nébulisation, de sorte que celui-ci est projeté desdites buses sous forme d'un jet à faible pression, lequel sous l'effet de la gravité, retombe intégralement sous la grille (31) du support (3), et est éventuellement intégralement reçu dans le bac de récupération (22) et l'ouverture d'évacuation (23) du châssis (2), et sans passer au travers des ouvertures (32) de ladite grille (31).

## Patentansprüche

1. Plattform (1) zum Desinfizieren der Schuhe (C) eines Benutzers (U) durch Sprühen einer Desinfektionsflüssigkeit, umfassend:
- einen Rahmen (2),
- einen Träger (3), der dazu ausgebildet ist, die Schuhe (C) eines Benutzers (U) aufzunehmen, und der fest mit dem Rahmen (2) verbunden ist,
- ein Ausstoßmittel (4) für Desinfektionsflüssigkeit, das dazu ausgebildet ist, Desinfektionsflüssigkeit auf die Schuhe (C) des Benutzers (U) zu sprühen, und das fest mit dem Rahmen (2) verbunden ist,
- eine Steuereinheit (5), die mit dem Ausstoßmittel (4) verbunden und dazu ausgebildet ist, das Sprühen von Desinfektionsflüssigkeit durch das Ausstoßmittel zu steuern (4),
wobei der Träger (3) ein Gitter (31) umfasst, das eine Mehrzahl von Öffnungen (32) aufweist, wobei das Gitter (31) dazu ausgebildet ist, die Unterseite (FI) der Sohlen (S) der Schuhe (C) des Benutzers (U) anliegend aufzunehmen, wobei das Ausstoßmittel (4) eine Mehrzahl von Düsen (41) aufweist, die unter dem Gitter (31) des Trägers (3) angeordnet und dazu ausgebildet sind, Desinfektionsflüssigkeit auf die Schuhe (C) des Benutzers (U) zu sprühen, durch wenigstens einen Teil der Öffnungen (32) des Gitters (31) des Trägers (3) in Form von Vernebelung in einer vorbestimmten, von der Steuereinheit (5) gesteuerten Menge,
wobei der Rahmen (2) im unteren Teil, unter dem Gitter (31) des Trägers (3) und unter den Düsen (41), eine Auffangwanne (22) aufweist,
wobei das Ausstoßmittel (4) einen Tank (42) für Desinfektionsflüssigkeit umfasst, der fest mit dem Rahmen (2) verbunden ist, und
wobei das Ausstoßmittel (4) eine einzige Pumpe (43) umfasst, die dazu ausgebildet ist, alle Düsen (41) gleichzeitig zu versorgen,
**dadurch gekennzeichnet, dass**
jede Düse (41) mit einem separaten Magnetventil (44) verbunden ist, das zwischen der Pumpe (43) und jeder der Düsen (41) angeordnet ist,
wobei die Steuereinheit dazu ausgebildet ist, das Sprühen von Desinfektionsflüssigkeit durch das Ausstoßmittel (4) zu steuern, indem sie einen Desinfektionszyklus mit dem Sprühen von Desinfektionsflüssigkeit in Form von Vernebelung auf die Schuhe (C) eines Benutzers (U) einleitet, mittels der Düsen (41) des Ausstoßmittels (4) durch die Öffnungen (32) des Gitters (31) des Trägers (3), auf dem die Unterseite (FI) der Sohlen (S) der Schuhe (C) des Benutzers (U) anliegend aufgenommen ist, und wobei der Schritt der Einleitung des Desinfektionszyklus die folgenden aufeinanderfolgenden Unterschritte umfasst:
- Voraktivierung der Pumpe (43), wobei die Magnetventile (44) für eine vorbestimmte Zeit zwischen 0,5 Sekunden und 2 Sekunden, bevorzugt etwa 1 Sekunde, geschlossen bleiben.
- Öffnen der Magnetventile (44) und Halten der aktivierten Pumpe (43) für eine Zeitdauer zwischen 2 Sekunden und 5 Sekunden, bevorzugt zwischen 3 und 4 Sekunden, um mittels der Düsen (41) Desinfektionsflüssigkeit durch die Öffnungen (32) des Gitters (31) des Trägers (3) zu sprühen,
und wobei die Durchflussmenge der Pumpe (43) viel geringer ist als die Durchflussmenge der Düsen (41), und wobei die Aktivierung der Pumpe (43) vor dem Öffnen der Magnetventile (44) für die vorbestimmte Zeitdauer zwischen 0,5 Sekunden und 2 Sekunden, bevorzugt etwa 1 Sekunde, dazu ausgebildet ist, den Versorgungskreislauf der Düsen (41) mit Druck zu beaufschlagen, so dass der Druck darin ausreichend ist, um das Sprühen von Desinfektionsflüssigkeit in Form einer Vernebelung zu gewährleisten, und während der genannten Pumpenaktivierungszeit zwischen 2 Sekunden und 5 Sekunden, bevorzugt zwischen 3 und 4 Sekunden, die gewünschte Durchflussmenge an Desinfektionsflüssigkeit zu den Düsen (41) zu liefern, beispielsweise zwischen 0,05 l/min und 1,2 l/min, bevorzugt zwischen 0,07 l/min und 0,09 l/min, und wobei im Falle einer Fehlfunktion nach dieser Pumpenaktivierungszeit zwischen 2 s und 4 s die von der Pumpe (43) gelieferte Durchflussmenge nicht ausreicht, damit die Düsen (41) Desinfektionsflüssigkeit in Form einer Vernebelung sprühen können, so dass diese aus den genannten Düsen in Form eines Niederdruckstrahls ausgestoßen wird, der unter dem Einfluss der Schwerkraft vollständig unter das Gitter (31) des Trägers (3) fällt und gegebenenfalls vollständig in der Auffangwanne (22) und der Auslassöffnung (23) des Rahmens (2) aufgenommen wird, und ohne durch die Öffnungen (32) des genannten Gitters (31) zu gelangen.

2. Plattform (1) nach Anspruch 1, wobei die Pumpe (43) dazu ausgebildet ist, eine Zufuhr an Desinfektionsflüssigkeit zwischen 7 I/Stunde und 20 l/Stunde, bevorzugt zwischen 10 l/Stunde und 15 l/Stunde, zu liefern.

3. Plattform (1) nach einem der Ansprüche 1 bis 2, wobei jede Düse (41) dazu ausgebildet ist, Desinfektionsflüssigkeit in Form von vernebelten Partikeln mit einem Durchmesser zwischen 40 µm und 300 µm, bevorzugt zwischen 80 µm und 150 µm, zu sprühen.

4. Plattform (1) nach einem der Ansprüche 1 bis 3, wobei jede Düse (41) dazu ausgebildet ist, Desinfektionsflüssigkeit in Form einer Vernebelung zu sprühen, wenn sie mit Desinfektionsflüssigkeit mit einer Durchflussmenge zwischen 0,05 l/min und 1 l/min, bevorzugt zwischen 0,07 l/min und 0,09 l/min, versorgt wird.

5. Plattform (1) nach einem der Ansprüche 1 bis 4, wobei das Ausstoßmittel (4) vier identische Düsen (41) umfasst, die zwei Düsenpaare (P41, P41') bilden, wobei ein erstes Düsenpaar (P41) derart positioniert ist, dass es Desinfektionsflüssigkeit auf die Unterseite (FI) der Sohle (S) eines ersten Schuhs (C) eines Benutzers (U) ausstößt, und ein zweites Düsenpaar (P41') so positioniert ist, dass es Desinfektionsflüssigkeit auf die Unterseite (FI) der Sohle (S) eines zweiten Schuhs (C) des Benutzers (U) ausstößt, und wobei insbesondere die Düsen (41) jedes Düsenpaars (P41, P41') einander gegenüberliegend angeordnet sind, und/oder die beiden Düsenpaare (P41, P41') zueinander symmetrisch sind.

6. Plattform (1) nach einem der Ansprüche 1 bis 5, wobei die Desinfektionsflüssigkeit aus einem Peroxid besteht und das Peroxid insbesondere Wasserstoffperoxid ist.

7. Plattform (1) nach einem der Ansprüche 1 bis 6, wobei die Öffnungen (32) des Gitters (31) wenigstens teilweise eine im Wesentlichen rechteckige Form mit einer Länge (L32) aufweisen, von in der Querrichtung (Y) der Plattform (1) zwischen 50 mm und 80 mm, bevorzugt zwischen 60 mm und 75 mm, und eine Breite (W32), von in der Längsrichtung (X) der Plattform (1) zwischen 50 mm und 80 mm, bevorzugt zwischen 60 mm und 75 mm, aufweisen, wobei zwei benachbarte Öffnungen (32) durch eine Materialdicke (W31) zwischen 3 mm und 6 mm, bevorzugt zwischen 4 mm und 5 mm, voneinander getrennt sind und insbesondere das Gitter (31) eine Mehrzahl von miteinander verschweißten Metalldrähten (F31) umfasst, die die Öffnungen (32) bilden, wobei der Durchmesser der Drähte zwischen 3 mm und 6 mm, bevorzugt zwischen 4 mm und 5 mm, liegt.

8. Plattform (1) nach einem der Ansprüche 1 bis 7, wobei der Träger (3) ein Trennelement (33) für die Füße eines Benutzers aufweist, das an dem Träger (3) befestigt ist, bevorzugt im Wesentlichen in der Mitte des Gitters (31), in der Querrichtung (Y) der Plattform (1), und dazu ausgebildet ist, auf dem Träger (3) in der Querrichtung (Y) der Plattform (1) einen ersten Raum (34) zu begrenzen, der dazu ausgebildet ist, den rechten Schuh (C) eines Benutzers (U) aufzunehmen, und einen zweiten Raum (35) zu begrenzen, der dazu ausgebildet ist, den linken Schuh (C) eines Benutzers (U) aufzunehmen, und insbesondere der Träger (3) umfasst:
- eine rechte Anschlagwand (34D), die dazu ausgebildet ist, in Kombination mit dem Trennelement (33) den ersten Raum (34) des Trägers (3) in der Querrichtung (Y) der Plattform (1) zu begrenzen und/oder
- eine rechte vordere Anschlagwand (34FD) und eine rechte hintere Anschlagwand (34RD), die dazu ausgebildet sind, den ersten Raum (34) des Trägers (3) in der Längsrichtung (X) der Plattform (1) vorne bzw. hinten zu begrenzen, und/oder
- eine linke Anschlagwand (35G), die dazu ausgebildet ist, in Kombination mit dem Trennelement (33) den zweiten Raum (35) des Trägers (3) in der Querrichtung (Y) der Plattform (1) zu begrenzen und/oder
- eine linke vordere Anschlagwand (35FG) und eine linke hintere Anschlagwand (35RG), die dazu ausgebildet sind, den zweiten Raum (35) des Trägers (3) in der Längsrichtung (X) der Plattform (1) vorne bzw. hinten zu begrenzen.

9. Plattform nach Anspruch 8, wobei ein erster Teil der Düsen (41) dazu ausgebildet ist, Desinfektionsflüssigkeit nur in den ersten Raum (34) des Trägers (3) zu sprühen, und ein zweiter Teil der Düsen (41) dazu ausgebildet ist, Desinfektionsflüssigkeit nur in den zweiten Raum (35) des Trägers (3) auszustoßen.

10. Plattform (1) nach einem der Ansprüche 1 bis 9, ferner umfassend eine mit der Steuereinheit (5) verbundene Einrichtung (6) zum Erfassen der Schuhe (C) eines Benutzers (U), die dazu ausgebildet ist, das Vorhandensein der Schuhe (C) eines Benutzers (U) auf dem Gitter (31) des Trägers (3) zu erfassen, wobei die Steuereinheit (5) dazu ausgebildet ist, das Sprühen von Desinfektionsflüssigkeit auf die Schuhe (C) eines Benutzers (U) durch die Ausstoßmittel (4) zu steuern, nachdem das Vorhandensein beider Schuhe (C) des Benutzers (U) auf dem Gitter (31) des Träger (3) erfasst wurde.

11. Plattform (1) nach Anspruch 10 in Kombination mit Anspruch 9, wobei das Erfassungsmittel (6) dazu ausgebildet ist, die Anwesenheit des rechten Schuhs (C) eines Benutzers (U) in dem ersten Raum (34) des Trägers (3) zu erfassen und die Anwesenheit des linken Schuhs (C) des Benutzers (U) in dem zweiten Raum (35) des Trägers (3) zu erfassen,
und wobei die Steuereinheit (5) dazu ausgebildet ist, das Sprühen von Desinfektionsflüssigkeit auf die Schuhe (C) eines Benutzers (U) durch das Ausstoßmittel (4) zu steuern, nachdem das Vorhandensein des rechten Schuhs (C) des Benutzers (U) in dem ersten Raum (34) des Trägers (3) und das Vorhandensein des linken Schuhs (C) des Benutzers (U) in dem zweiten Raum (35) des Trägers (3) erfasst wurde.

12. Plattform (1) nach Anspruch 10 oder 11, wobei das Erfassungsmittel (6) ein Mittel zum Senden (61) eines Signals, beispielsweise eines Lichtsignals, bevorzugt eines Infrarotsignals, und ein Mittel zum Empfangen (62) des von dem Sendemittel (61) gesendeten Signals umfasst.

13. Plattform (1) nach Anspruch 12 in Verbindung mit Anspruch 11, wobei das Erfassungsmittel (6) umfasst:
- ein erstes Sendemittel (61), das dazu ausgebildet ist, ein Signal in den ersten Raum (34) des Träger (3) zu senden, und ein erstes Empfangsmittel (62), das dazu ausgebildet ist, das von dem ersten Sendemittel (61) gesendete Signal zu empfangen, und
- ein zweites Sendemittel (61), das dazu ausgebildet ist, ein Signal in den zweiten Raum (35) des Trägers (3) zu senden, und ein zweites Empfangsmittel (62), das dazu ausgebildet ist, das vom zweiten Sendemittel (61) gesendete Signal zu empfangen.

14. Verfahren zum Desinfizieren der Schuhe (C) eines Benutzers (U), das mit einer Plattform (1) nach einem der Ansprüche 1 bis 13 durchgeführt wird, umfassend den folgenden Schritt:
- Starten eines Desinfektionszyklus mit Sprühen von Desinfektionsflüssigkeit in Form einer Vernebelung auf die Schuhe (C) eines Benutzers (U) aus den Düsen (41) des Ausstoßmittels (4) durch die Öffnungen (32) des Gitters (31) des Trägers (3), auf dem die Unterseite (FI) der Sohlen (S) der Schuhe (C) des Benutzers (U) anliegend aufgenommen wird.

15. Desinfektionsverfahren nach Anspruch 14 in Kombination mit einem der Ansprüche 10 bis 13, welches ferner vor dem Schritt des Startens des Desinfektionszyklus einen Schritt des Erfassens des Vorhandenseins der Schuhe (C) eines Benutzers (U) auf dem Gitter (31) des Trägers (3) umfasst, wobei der Schritt des Startens des Desinfektionszyklus nur dann ausgeführt wird, wenn das Vorhandensein der Schuhe (C) des Benutzers (U) auf dem Gitter (31) des Trägers (3) erfasst wird.

16. Desinfektionsverfahren nach Anspruch 14 oder 15 in Verbindung mit den Ansprüchen 1 und 3, wobei der Schritt zum Starten des Desinfektionszyklus die folgenden aufeinanderfolgenden Teilschritte umfasst:
- Voraktivierung der Pumpe (43), wobei die Magnetventile (44) für eine vorbestimmte Zeitdauer zwischen 0,5 Sekunden und 2 Sekunden, bevorzugt etwa 1 Sekunde, geschlossen bleiben.
- Öffnen der Magnetventile (44) und Halten der aktivierten Pumpe (43) für eine Zeitdauer zwischen 2 Sekunden und 5 Sekunden, bevorzugt zwischen 3 und 4 Sekunden, so dass Desinfektionsflüssigkeit durch die Öffnungen (32) des Gitters (31) des Trägers (3) mittels der Düsen (41) gesprüht wird, und wobei die Durchflussmenge der Pumpe (43) viel geringer ist als die Durchflussmenge der Düsen (41), und wobei die Aktivierung der Pumpe (43) vor dem Öffnen der Magnetventile (44) für die vorbestimmte Zeitdauer zwischen 0,5 Sekunden und 2 Sekunden, bevorzugt etwa 1 Sekunde, ausgebildet ist, um den Versorgungskreislauf der Düsen (41) mit Druck zu beaufschlagen, so dass der Druck darin ausreichend ist, um das Sprühen der Desinfektionsflüssigkeit in Form einer Vernebelung zu gewährleisten, und während der genannten Zeitdauer der Pumpenaktivierung zwischen 2 Sekunden und 5 Sekunden, bevorzugt zwischen 3 und 4 Sekunden, indem den Düsen (41) die gewünschte Durchflussmenge an Desinfektionsflüssigkeit zugeführt wird, beispielsweise zwischen 0,05 l/min und 1,2 l/min, bevorzugt zwischen 0,07 l/min und 0,09 l/min, und wobei im Falle einer Fehlfunktion nach dieser Pumpenaktivierungszeit zwischen 2 s und 4 s die von der Pumpe (43) zugeführte Menge nicht ausreicht, damit die Düsen (41) Desinfektionsflüssigkeit in Form einer Vernebelung versprühen können, so dass diese aus den Düsen in Form eines Niederdruckstrahls ausgestoßen wird, der unter dem Einfluss der Schwerkraft vollständig unter das Gitter (31) des Trägers (3) fällt und gegebenenfalls vollständig in der Auffangwanne (22) und der Auslassöffnung (23) des Rahmens (2) aufgenommen wird, und ohne durch die Öffnungen (32) des genannten Gitters (31) zu gelangen.

## Claims

1. A platform (1) for disinfecting the shoes (C) of a user (U) by spraying a disinfectant liquid, including:
- a frame (2),
- a support (3) configured to receive the shoes (C) of a user (U), and integral with the frame (2),
- a disinfectant liquid projection means (4), configured to project disinfectant liquid onto the shoes (C) of the user (U), and integral with the frame (2),
- a control unit (5) connected to the projection means (4), and configured so as to control the projection of disinfectant liquid by the projection means (4),
wherein the support (3) includes a grid (31) having a plurality of openings (32), the grid (31) being configured so as to receive in support the lower face (F1) of the soles (S) of the shoes (C) of the user (U), wherein the projection means (4) has a plurality of nozzles (41), disposed under the grid (31) of the support (3), and configured so as to project disinfectant liquid onto the shoes (C) of the user (U) through at least part of the openings (32) of the grid (31) of the support (3), in the form of a nebulisation, according to a determined amount, controlled by the control unit (5),
wherein the frame (2) includes in the lower part, under the grid (31) of the support (3) and under the nozzles (41), a recovery tank (22),
wherein the projection means (4) includes a reservoir (42) of disinfectant liquid, integral with the frame (2), and
wherein the projection means (4) includes a single pump (43) configured so as to simultaneously supply all the nozzles (41), **characterised in that** each nozzle (41) is connected to a separate solenoid valve (44), interposed between the pump (43) and each of the nozzles (41),
wherein said control unit is configured so as to control the projection of disinfectant liquid by the projection means (4), by starting a disinfection cycle with projection of disinfectant liquid in the form of a nebulisation onto the shoes (C) of a user (U), by the nozzles (41) of the projection means (4) through the openings (32) of the grid (31) of the support (3), on which the lower face (FI) of the soles (S) of the shoes (C) of the user (U) is supported and wherein the step of launching the disinfection cycle including the successive sub-steps:
- prior activation of the pump (43) with the solenoid valves (44) remaining closed for a predetermined duration comprised between 0.5 seconds and 2 seconds, preferably approximately 1 second,
- opening of the solenoid valves (44) and maintenance of the pump (43) activated for a duration comprised between 2 seconds and 5 seconds, preferably between 3 and 4 seconds, so as to project disinfectant liquid through the openings (32) of the grid (31) of the support (3) via the nozzles (41), and wherein the flow rate of the pump (43) is much lower than the flow rate of the nozzles (41), and wherein the activation of the pump (43) prior to the opening of the solenoid valves (44) for the predetermined duration, comprised between 0.5 seconds and 2 seconds, preferably approximately 1 second, is configured to pressurise the supply circuit of the nozzles (41), so that the pressure therein is sufficient to ensure the projection of disinfectant liquid in the form of a nebulisation and during said pump activation duration comprised between 2 seconds and 5 seconds, preferably between 3 and 4 seconds, providing the desired disinfectant liquid supply flow rate to the nozzles (41), for example comprised between 0.05 L/min and 1.2 L/min, preferably between 0.07 L/min and 0.09 L/min, and wherein in the event of a malfunction, beyond this activation duration of the pump comprised between 2s and 4s, the flow rate provided by the pump (43) is insufficient for the nozzles 41 to be able to project disinfectant liquid in the form of a nebulisation, so that it is projected from said nozzles in the form of a low pressure jet, which under the effect of gravity, falls entirely under the grid (31) of the support (3), and is possibly entirely received in the recovery tank (22) and the evacuation opening (23) of the frame (2), and without passing through the openings (32) of said grid (31).

2. The platform (1) according to claim 1, wherein the pump (43) is configured so as to provide a flow rate of disinfectant liquid comprised between 7L/hour and 20 L/hour, preferably comprised between 10 L/hour and 15 L/hour.

3. The platform (1) according to one of claims 1 to 2, wherein each nozzle (41) is configured to project disinfectant liquid in the form of a nebulisation of particles with a diameter comprised between 40 µm and 300 µm, preferably between 80 pm and 150 pm.

4. The platform (1) according to one of claims 1 to 3, wherein each nozzle (41) is configured to project disinfectant liquid in the form of a nebulisation when supplied with disinfectant liquid with a flow rate comprised between 0.05 L/min and 1.2 L/min, preferably between 0.07 L/min and 0.09 L/min.

5. The platform (1) according to one of claims 1 to 4, wherein the projection means (4) comprises four identical nozzles (41), forming two pairs of nozzles (P41, P41'), with a first pair of nozzles (P41) positioned so as to project disinfectant liquid onto the lower face (FI) of the sole (S) of a first shoe (C) of a user (U), and a second pair of nozzles (P41') positioned so as to project disinfectant liquid onto the lower face (FI) of the sole (S) of a second shoe (C) of the user (U) and wherein in particular the nozzles (41) of each pair of nozzles (P41, P41') are disposed opposite each other, and/or the two pairs of nozzles (P41, P41') are symmetrical to each other.

6. The platform (1) according to one of claims 1 to 5, wherein the disinfectant liquid consists of a peroxide, and in particular the peroxide is hydrogen peroxide.

7. The platform (1) according to one of claims 1 to 6, wherein the openings (32) of the grid (31) have at least for part of them a substantially rectangular shape of length (L32), in the transverse direction (Y) of the platform (1), comprised between 50 mm and 80 mm, preferably comprised between 60 mm and 75 mm, and of width (W32), in the longitudinal direction (X) of the platform (1), comprised between 50 mm and 80 mm, preferably between 60 mm and 75 mm, two adjacent openings (32) being separated from each other by a thickness of material (W31) comprised between 3 mm and 6 mm, preferably comprised between 4 mm and 5 mm and in particular the grid (31) includes a plurality of metal wires (F31) welded together, forming said openings (32), the diameter of the wires being comprised between 3 mm and 6 mm, preferably comprised between 4 mm and 5mm.

8. The platform (1) according to one of claims 1 to 7, wherein the support (3) includes an element (33) for separating the feet of a user, fixed to the support (3), preferably substantially at the middle of the grid (31), in the transverse direction (Y) of the platform (1), and configured so as to delimit on the support (3), in the transverse direction (Y) of the platform (1), a first space (34) configured to receive the right shoe (C) of a user (U) and a second space (35) configured to receive the left shoe (C) of a user (U) and in particular the support (3) includes:
- a right abutment wall (34D), configured to delimit in combination with the separating element (33) the first space (34) of the support (3), in the transverse direction (Y) of the platform (1), and/or
- a right front abutment wall (34FD) and a right rear abutment wall (34RD), configured to delimit the first space (34) of the support (3), in the longitudinal direction (X) of the platform (1), at the front and rear respectively, and/or
- a left abutment wall (35G), configured to delimit in combination with the separating element (33) the second space (35) of the support (3), in the transverse direction (Y) of the platform (1), and/or
- a left front abutment wall (35FG) and a left rear abutment wall (35RG), configured to delimit the second space (35) of the support (3), in the longitudinal direction (X) of the platform (1), at the front and rear respectively.

9. The platform according to claim 8, wherein a first part of the nozzles (41) are configured so as to project disinfectant liquid only into the first space (34) of the support (3) and a second part of the nozzles (41) are configured so as to project disinfectant liquid only into the second space (35) of the support (3).

10. The platform (1) according to one of claims 1 to 9, further comprising a means (6) for detecting the shoes (C) of a user (U), connected to the control unit (5), configured so as to detect the presence of the shoes (C) of a user (U) on the grid (31) of the support (3), the control unit (5) being configured to control the projection of disinfectant liquid onto the shoes (C) of a user (U) by the projection means (4) after the detection of the presence of the two shoes (C) of the user (U) on the grid (31) of the support (3).

11. The platform (1) according to claim 10 as combined with claim 9 wherein the detection means (6) is configured to detect the presence of the right shoe (C) of a user (U) in the first space (34) of the support (3) and to detect the presence of the left shoe (C) of the user (U) in the second space (35) of the support (3), and wherein the control unit (5) is configured to control the projection of disinfectant liquid onto the shoes (C) of a user (U) by the projection means (4) after the detection of the presence of the right shoe (C) of the user (U) in the first space (34) of the support (3) and the presence of the left shoe (C) of the user (U) in the second space (35) of the support (3).

12. The platform (1) according to claim 10 or 11, wherein the detection means (6) includes means for emitting (61) a signal, for example a light signal, preferably infrared, and a means for receiving (62) the signal emitted by the emission means (61).

13. The platform (1) according to claim 12 as combined with claim 11, wherein the detection means (6) includes:
- a first emission means (61), configured to emit a signal in the first space (34) of the support (3), and a first reception means (62), configured to receive the signal emitted by the first emission means (61), and
- a second emission means (61), configured to emit a signal in the second space (35) of the support (3), and a second reception means (62), configured to receive the signal emitted by the second emission means (61).

14. A method for disinfecting the shoes (C) of a user (U) implemented with a platform (1) according to one of claims 1 to 13, comprising the step:
- launching a disinfection cycle with projection of disinfectant liquid in the form of a nebulisation onto the shoes (C) of a user (U), by the nozzles (41) of the projection means (4) through the openings (32) of the grid (31) of the support (3), on which the lower face (FI) of the soles (S) of the shoes (C) of the user (U) is supported.

15. The disinfection method according to claim 14 as combined with one of claims 10 to 13, further comprising, prior to the step of launching the disinfection cycle, a step of detecting the presence of shoes (C) of a user (U) on the grid (31) of the support (3), the step of launching the disinfection cycle being executed only if the presence of the shoes (C) of the user (U) on the grid (31) of the support (3) is detected.

16. The disinfection method according to claim 14 or 15, as combined with claims 1 and 3, wherein, the step of launching the disinfection cycle includes the successive sub-steps:
- prior activation of the pump (43) with the solenoid valves (44) remaining closed for a predetermined duration comprised between 0.5 seconds and 2 seconds, preferably approximately 1 second,
- opening of the solenoid valves (44) and maintenance of the pump (43) activated for a duration comprised between 2 seconds and 5 seconds, preferably between 3 and 4 seconds, so as to project disinfectant liquid through the openings (32) of the grid (31) of the support (3) via the nozzles (41), and wherein the flow rate of the pump (43) is much lower than the flow rate of the nozzles (41), and wherein the activation of the pump (43) prior to the opening of the solenoid valves (44) for the predetermined duration, comprised between 0.5 seconds and 2 seconds, preferably approximately 1 second, is configured to pressurise the supply circuit of the nozzles (41), so that the pressure therein is sufficient to ensure the projection of disinfectant liquid in the form of a nebulisation and during said pump activation duration comprised between 2 seconds and 5 seconds, preferably between 3 and 4 seconds, providing the desired disinfectant liquid supply flow rate to the nozzles (41), for example comprised between 0.05 L/min and 1.2 L/min, preferably between 0.07 L/min and 0.09 L/min, and wherein in the event of a malfunction, beyond this activation duration of the pump comprised between 2s and 4s, the flow rate provided by the pump (43) is insufficient for the nozzles 41 to be able to project disinfectant liquid in the form of a nebulisation, so that it is projected from said nozzles in the form of a low pressure jet, which under the effect of gravity, falls entirely under the grid (31) of the support (3), and is possibly entirely received in the recovery tank (22) and the evacuation opening (23) of the frame (2), and without passing through the openings (32) of said grid (31).
